# EUROPEAN PATENT APPLICATION

(11) **EP 4 431 606 A1**
(43) Date of publication of application: **18.09.2024**
(21) Application number: 22892818.0
(22) Date of filing: 09.11.2022
(51) Int. Cl.: C12N 15/13, C07K 16/28, C07K 16/40, C12N 1/15, C12N 1/19, C12N 1/21, C12N 5/10, C12N 15/63, C12P 21/08, G01N 33/531, G01N 33/573

(54) **ANTI-EPHA4 ANTIBODY**

(30) Priority: 11.11.2021 JP 2021184126
(71) Applicant: Eisai R&D Management Co., Ltd., Tokyo 112-8088 (JP)
(72) Inventor: KAWAKATSU, Tomomi, Kobe-shi, Hyogo 650-0047 (JP); TAHARA, Kazuhiro, Tsukuba-shi, Ibaraki 300-2635 (JP); SHUTA, Kazuyoshi, Tsukuba-shi, Ibaraki 300-2635 (JP)
(74) Representative: Hoffmann Eitle
(86) International application number: PCT/JP2022/041723
(87) International publication number: WO 2023/085320

(57) **Abstract**

[Problem] To provide an antibody that can bind specifically to EphA4 and detect EphA4 at high detection sensitivity, and a method and a kit for detecting or quantifying EphA4 characterized by the antibody.

[Solution] Provided are an antibody having a specific heavy chain CDR sequence and light chain CDR sequence and an antigen binding fragment thereof, and a method and kit characterized by the same. Specifically, an antibody according to the present invention or an antigen-binding fragment thereof includes (a) a heavy chain including a heavy chain CDR1 comprising an amino acid sequence represented by SEQ ID NO. 52; a heavy chain CDR2 comprising an amino acid sequence represented by SEQ ID NO. 53; and a heavy chain CDR3 comprising an amino acid sequence represented by SEQ ID NO. 54; and a light chain including a light chain CDR1 comprising an amino acid sequence represented by SEQ ID NO. 55; a light chain CDR2 comprising an amino acid sequence represented by SEQ ID NO. 56; and a light chain CDR3 comprising an amino acid sequence represented by SEQ ID NO. 57; (b) a heavy chain including a heavy chain CDR1 comprising an amino acid sequence represented by SEQ ID NO. 64; a heavy chain CDR2 comprising an amino acid sequence represented by SEQ ID NO. 65; and a heavy chain CDR3 comprising an amino acid sequence represented by SEQ ID NO. 66; and a light chain including a light chain CDR1 comprising an amino acid sequence presented by SEQ ID NO. 67; a light chain CDR2 comprising an amino acid sequence represented by SEQ ID NO. 68; and a light chain CDR3 comprising an amino acid sequence represented by SEQ ID NO. 69; or(c) a heavy chain including a heavy chain CDR1 comprising an amino acid sequence represented by SEQ ID NO. 40; a heavy chain CDR2 comprising an amino acid sequence represented by SEQ ID NO. 41; and a heavy chain CDR3 comprising an amino acid sequence represented by SEQ ID NO. 42; and a light chain including a light chain CDR1 comprising an amino acid sequence represented by SEQ ID NO. 43; a light chain CDR2 comprising an amino acid sequence represented by SEQ ID NO. 44; and a light chain CDR3 comprising an amino acid sequence represented by SEQ ID NO. 45.

## Description

### Technical Field

The present disclosure relates to an antibody that binds to EphA4, a nucleic acid encoding the antibody, a vector comprising the nucleic acid, a cell comprising the vector, a method for producing the antibody, as well as an EphA4 detection or quantification method employing the antibody and a kit for detecting or quantifying EphA4.

### Background Art

EphA4 is a member of the receptor-type tyrosine kinase family, and is a molecule that controls spines which are small spiney structures present on dendrites. Ephrin type A and type B are known as ligands of EphA4, when EphA4 binds to its ligand ephrin, deadhesion signal is induced, and causes retraction of spines. EphA4 is highly expressed in the hippocampus or the cerebral cortex, and its extracellular domain is cleaved by matrix metalloprotease (MMP) or ADAM (a disintegrin and metalloproteinase) . These cleaved EphA4 fragments are released extracellularly, and also exist in plasma (Patent Literature 1).

EphA4 has been hitherto suggested to be involved in the pathology of Alzheimer's disease (hereinafter also referred to as "AD") (Non-Patent Literatures 1 to 4) . For example, EphA4 is known to be activated in AD patients or AD model mice (Non-Patent Literatures 2 to 4), and since spine density decreases in AD and the extent thereof is related to clinical symptoms of AD (Non-Patent Literature 5), it is thought that abnormal EphA4 activation may be one cause of onset or progression of pathology of AD (Non-Patent Literature 6). EphA4 in vivo has been suggested to have potential as a marker that may detect given nervous system diseases such as AD, and antibodies that can detect EphA4 or extracellular fragments thereof in biological samples at a higher detection sensitivity than conventional antibodies are desired.

### Citation List

[Patent Literature 1] WO 2012/147798A1

[Non-Patent Literature 1] Rosenberger AF et al., Acta Neuropathol Commun. 2014 Jul 16; 2:79
[Non-Patent Literature 2] Fu AK et al., Proc Natl Acad Sci USA. 2014 Jul 8; 111(27):9959-64
[Non-Patent Literature 3] Vargas LM et al., PLoS One. 2014 Mar 21; 9(3)
[Non-Patent Literature 4] Huang TY et al., J Exp Med. 2017 Dec 4; 214(12) :3669-3685.
[Non-Patent Literature 5] Boros et al., Ann Neurol. 2017 Oct; 82 (4) : 602-614
[Non-Patent Literature 6] Vargas LM et al., Biochim Biophys Acta Mol Basis Dis. 2018 Apr; 1864:1148-1159

### Summary of the Invention

### Problems to be Solved by the Invention

The object of the present disclosure is to provide an antibody that can specifically bind to EphA4 and detect EphA4 with high detection sensitivity.

The object of the present disclosure is also to provide a method for detecting or quantifying EphA4 employing the antibody.

The object of the present disclosure is further to provide a kit comprising an anti-EphA4 antibody that can specifically detect or quantify EphA4 with high detection sensitivity.

### Means for Solving the Problems

In order to solve the above problems, the present inventors found that antibodies that can specifically bind to EphA4 with particularly high detection sensitivity can be formed from numerous scFvs obtained by screening rabbit antibody phage libraries, thus coming to complete the anti-EphA4 antibody.

Accordingly, the present disclosure encompasses the following characteristics.
[1] An anti-EphA4 antibody or an antigen binding fragment thereof, wherein the antibody comprises
   (a) a heavy chain comprising a heavy chain CDR1 consisting of the amino acid sequence shown in SEQ ID NO. 52; a heavy chain CDR2 consisting of the amino acid sequence shown in SEQ ID NO. 53; and a heavy chain CDR3 consisting of the amino acid sequence shown in SEQ ID NO. 54; and
      a light chain comprising a light chain CDR1 consisting of the amino acid sequence shown in SEQ ID NO. 55; a light chain CDR2 consisting of the amino acid sequence shown in SEQ ID NO. 56; and a light chain CDR3 consisting of the amino acid sequence shown in SEQ ID NO. 57;
   (b) a heavy chain comprising a heavy chain CDR1 consisting of the amino acid sequence shown in SEQ ID NO. 64; a heavy chain CDR2 consisting of the amino acid sequence shown in SEQ ID NO. 65; and a heavy chain CDR3 consisting of the amino acid sequence shown in SEQ ID NO. 66; and
      a light chain comprising a light chain CDR1 consisting of the amino acid sequence shown in SEQ ID NO. 67; a light chain CDR2 consisting of the amino acid sequence shown in SEQ ID NO. 68; and a light chain CDR3 consisting of the amino acid sequence shown in SEQ ID NO. 69; or
   (c) a heavy chain comprising a heavy chain CDR1 consisting of the amino acid sequence shown in SEQ ID NO. 40; a heavy chain CDR2 consisting of the amino acid sequence shown in SEQ ID NO. 41; and a heavy chain CDR3 consisting of the amino acid sequence shown in SEQ ID NO. 42; and
      a light chain comprising a light chain CDR1 consisting of the amino acid sequence shown in SEQ ID NO. 43; a light chain CDR2 consisting of the amino acid sequence shown in SEQ ID NO. 44; and a light chain CDR3 consisting of the amino acid sequence shown in SEQ ID NO. 45.
[2] The anti-EphA4 antibody or antigen binding fragment thereof according to [1], wherein the antibody comprises
   a heavy chain comprising a heavy chain CDR1 consisting of the amino acid sequence shown in SEQ ID NO. 52; a heavy chain CDR2 consisting of the amino acid sequence shown in SEQ ID NO. 53; and a heavy chain CDR3 consisting of the amino acid sequence shown in SEQ ID NO. 54; and
   a light chain comprising a light chain CDR1 consisting of the amino acid sequence shown in SEQ ID NO. 55; a light chain CDR2 consisting of the amino acid sequence shown in SEQ ID NO. 56; and a light chain CDR3 consisting of the amino acid sequence shown in SEQ ID NO. 57.
[3] The anti-EphA4 antibody or antigen binding fragment thereof according to [2], wherein the antibody comprises
   a heavy chain variable region consisting of the amino acid sequence shown in SEQ ID NO. 10, and
   a light chain variable region consisting of the amino acid sequence shown in SEQ ID NO. 11.
[4] The anti-EphA4 antibody or antigen binding fragment thereof according to [2], wherein the antibody comprises
   a heavy chain variable region consisting of the amino acid sequence shown in SEQ ID NO. 14, and
   a light chain variable region consisting of the amino acid sequence shown in SEQ ID NO. 15.
[5] The anti-EphA4 antibody or antigen binding fragment thereof according to [1], wherein the antibody comprises
   a heavy chain comprising a heavy chain CDR1 consisting of the amino acid sequence shown in SEQ ID NO. 64; a heavy chain CDR2 consisting of the amino acid sequence shown in SEQ ID NO. 65; and a heavy chain CDR3 consisting of the amino acid sequence shown in SEQ ID NO. 66; and
   a light chain comprising a light chain CDR1 consisting of the amino acid sequence shown in SEQ ID NO. 67; a light chain CDR2 consisting of the amino acid sequence shown in SEQ ID NO. 68; and a light chain CDR3 consisting of the amino acid sequence shown in SEQ ID NO. 69.
[6] The anti-EphA4 antibody or antigen binding fragment thereof according to [5], wherein the antibody comprises
   a heavy chain variable region consisting of the amino acid sequence shown in SEQ ID NO. 18, and
   a light chain variable region consisting of the amino acid sequence shown in SEQ ID NO. 19.
[7] The anti-EphA4 antibody or antigen binding fragment thereof according to [1], wherein the antibody comprises
   a heavy chain comprising a heavy chain CDR1 consisting of the amino acid sequence shown in SEQ ID NO. 40; a heavy chain CDR2 consisting of the amino acid sequence shown in SEQ ID NO. 41; and a heavy chain CDR3 consisting of the amino acid sequence shown in SEQ ID NO. 42; and
   a light chain comprising a light chain CDR1 consisting of the amino acid sequence shown in SEQ ID NO. 43; a light chain CDR2 consisting of the amino acid sequence shown in SEQ ID NO. 44; and a light chain CDR3 consisting of the amino acid sequence shown in SEQ ID NO. 45.
[8] The anti-EphA4 antibody or antigen binding fragment thereof according to [7], wherein the antibody comprises
   a heavy chain variable region consisting of the amino acid sequence shown in SEQ ID NO. 6, and
   a light chain variable region consisting of the amino acid sequence shown in SEQ ID NO. 7.
[9] The anti-EphA4 antibody or antigen binding fragment thereof according to any of [1] to [8], wherein the heavy chain constant region of the antibody is the constant region of rabbit IgG.
[10] The anti-EphA4 antibody or antigen binding fragment thereof according to [9], wherein the constant region of rabbit IgG comprises the amino acid sequence shown in SEQ ID NO. 22.
[11] The anti-EphA4 antibody or antigen binding fragment thereof according to any of [1] to [10], wherein the light chain constant region of the antibody is the constant region of rabbit Igκ.
[12] The anti-EphA4 antibody or antigen binding fragment thereof according to [11], wherein the constant region of rabbit Igκ comprises the amino acid sequence shown in SEQ ID NO. 23.
[13] An anti-EphA4 antibody, wherein the antibody comprises
   a heavy chain comprising the amino acid sequence shown in SEQ ID NO. 24, and
   a light chain comprising the amino acid sequence shown in SEQ ID NO. 25.
[14] An anti-EphA4 antibody, wherein the antibody comprises
   a heavy chain comprising the amino acid sequence shown in SEQ ID NO. 28, and
   a light chain comprising the amino acid sequence shown in SEQ ID NO. 29.
[15] An anti-EphA4 antibody, wherein the antibody comprises
   a heavy chain comprising the amino acid sequence shown in SEQ ID NO. 32, and
   a light chain comprising the amino acid sequence shown in SEQ ID NO. 33.
[16] An anti-EphA4 antibody, wherein the antibody comprises
   a heavy chain comprising the amino acid sequence shown in SEQ ID NO. 36, and
   a light chain comprising the amino acid sequence shown in SEQ ID NO. 37.
[17] The anti-EphA4 antibody or antigen binding fragment thereof according to any of [1] to [16], wherein the antibody or an antigen binding fragment thereof is labeled.
[18] An isolated nucleic acid encoding the anti-EphA4 antibody or antigen binding fragment thereof according to any of [1] to [16].
[19] A vector comprising the nucleic acid according to [18].
[20] A host cell comprising the vector according to [19].
[21] A method for producing an anti-EphA4 antibody or an antigen binding fragment thereof, comprising a step of culturing the host cell according to [20].
[22] A method for producing an anti-EphA4 antibody or an antigen binding fragment thereof, comprising a step of culturing a host cell comprising an isolated nucleic acid encoding the anti-EphA4 antibody or antigen binding fragment thereof according to any of [1] to [16] .
[23] A method for detecting or quantifying human EphA4 in a biological sample, comprising contacting the biological sample with the anti-EphA4 antibody or antigen binding fragment thereof according to any of [1] to [16].
[24] The method according to [23], wherein the human EphA4 is the N-terminal fragment of human EphA4.
[25] The method according to [23] or [24], wherein the biological sample is blood, serum, plasma, or cerebrospinal fluid.
[26] The method according to any of [23] to [25], wherein the method is ELISA.
[27] The method according to any of [23] to [26], wherein the method is sandwich ELISA.
[28] The method according to any of [23] to [27], further comprising contacting the biological sample with the labeled anti-EphA4 antibody or antigen binding fragment thereof according to [17].
[29] A kit for detecting or quantifying human EphA4, comprising the anti-EphA4 antibody or antigen binding fragment thereof according to any of [1] to [17].
[30] The kit according to [29], wherein
   the kit is a sandwich ELISA kit, and
   comprises at least two types of the anti-EphA4 antibodies or antigen binding fragments thereof.
[31] The kit according to [29] or [30], comprising
   an anti-EphA4 antibody or an antigen binding fragment thereof comprising a heavy chain variable region consisting of the amino acid sequence shown in SEQ ID NO. 14 and a light chain variable region consisting of the amino acid sequence shown in SEQ ID NO. 15, and
   an anti-EphA4 antibody or an antigen binding fragment thereof comprising a heavy chain variable region consisting of the amino acid sequence shown in SEQ ID NO. 18 and a light chain variable region consisting of the amino acid sequence shown in SEQ ID NO. 19.
[32] The kit according to [29] or [30], comprising
   an anti-EphA4 antibody or an antigen binding fragment thereof comprising a heavy chain variable region consisting of the amino acid sequence shown in SEQ ID NO. 6 and a light chain variable region consisting of the amino acid sequence shown in SEQ ID NO. 7, and
   an anti-EphA4 antibody or an antigen binding fragment thereof comprising a heavy chain variable region consisting of the amino acid sequence shown in SEQ ID NO. 14 and a light chain variable region consisting of the amino acid sequence shown in SEQ ID NO. 15.
[33] The kit according to [29] or [30], comprising
   an anti-EphA4 antibody or an antigen binding fragment thereof comprising a heavy chain variable region consisting of the amino acid sequence shown in SEQ ID NO. 10 and a light chain variable region consisting of the amino acid sequence shown in SEQ ID NO. 11, and
   an anti-EphA4 antibody or an antigen binding fragment thereof comprising a heavy chain variable region consisting of the amino acid sequence shown in SEQ ID NO. 18 and a light chain variable region consisting of the amino acid sequence shown in SEQ ID NO. 19.
[34] The kit according to any of [29] to [33], wherein the human EphA4 is the N-terminal fragment of human EphA4.
[35] The kit according to any of [29] to [34], wherein the kit comprises the N-terminal fragment of human EphA4.
[36] The kit according to any of [29] to [35] for detecting or quantifying human EphA4 in a biological sample.
[37] The kit according to [36], wherein the biological sample is blood, serum, plasma, or cerebrospinal fluid.
[38] The anti-EphA4 antibody or antigen binding fragment thereof according to any of [1] to [17] for use in the detection or quantification of human EphA4.
[39] The anti-EphA4 antibody or antigen binding fragment thereof according to any of [1] to [17] for use in the detection or quantification of the N-terminal fragment of human EphA4.

### Effects of the Invention

According to the present disclosure, an antibody that can specifically bind to EphA4 and detect EphA4 with high detection sensitivity is provided.

Also according to the present disclosure, a kit comprising an anti-EphA4 antibody that can specifically detect EphA4 with high detection sensitivity is provided.

### Brief Description of the Drawings

Figure 1 shows the evaluation result of the binding activity of each anti-human EphA4 monoclonal antibody produced in Example 1 against each human Eph receptor family.
Figure 2 shows the evaluation result of the binding activity of each anti-human EphA4 monoclonal antibody produced in Example 1 against various EphA4.
Figure 3 shows the evaluation result of the binding activity of each anti-human EphA4 monoclonal antibody produced in Example 1 against each domain of human EphA4.
Figure 4 shows the evaluation result against human EphA4 extracellular domain S-N ((signal obtained when 10 ng/mL of EphA4 extracellular domain was seeded) - (signal obtained when 0 ng/mL of EphA4 extracellular domain was seeded)) against human EphA4 extracellular domain in sandwich ELISA employing combinations of anti-human EphA4 monoclonal antibodies produced in Example 1.
Figure 5 shows the evaluation result against human EphA4 extracellular domain S/N ((signal obtained when 10 ng/mL of EphA4 extracellular domain was seeded) / (signal obtained when 0 ng/mL of EphA4 extracellular domain was seeded)) against human EphA4 extracellular domain in sandwich ELISA employing combinations of anti-human EphA4 monoclonal antibodies produced in Example 1.
Figure 6 shows the evaluation result against human EphA4 extracellular domain S-N ((signal obtained when 1 ng/mL of EphA4 extracellular domain was seeded) - (signal obtained when 0 ng/mL of EphA4 extracellular domain was seeded)) against human EphA4 extracellular domain in sandwich ELISA employing combinations of anti-human EphA4 monoclonal antibodies produced in Example 1.
Figure 7 shows the evaluation result against human EphA4 extracellular domain S/N ((signal obtained when 1 ng/mL of EphA4 extracellular domain was seeded) / (signal obtained when 0 ng/mL of EphA4 extracellular domain was seeded))against human EphA4 extracellular domain in sandwich ELISA employing combinations of anti-human EphA4 monoclonal antibodies produced in Example 1.
Figure 8 shows the representative binding reaction curve of each antibody of KPEP11_04, KPEP11_08, KPEP11_10, and KPEP11_18 against human EphA4.
Figure 9 shows the binding specificity of each antibody of KPEP11_04, KPEP11_08, KPEP11_10, and KPEP11_18 against each human Eph receptor family.
Figure 10 shows the reactivity result of each antibody of KPEP11_04, KPEP11_08, KPEP11_10, and KPEP11_18 against mouse, rat, rabbit, monkey, and human EphA4.
Figure 11 shows the reactivity result of each antibody of KPEP11_04, KPEP11_08, KPEP11_10, and KPEP11_18 against the extracellular domain (ECD), ligand binding domain (LBD), fibronectin III-type domain 1 (FN1) and fibronectin III-type domain 2 (FN2) of human EphA4, and maltose-binding protein (MBP).
Figure 12 shows the reactivity result of sandwich ELISA employing antibody KPEP11_10 and HRP-labeled antibody KPEP11_18 against human EphA4 extracellular domain.
Figure 13 shows the reactivity result of sandwich ELISA employing antibody KPEP11_10 and HRP-labeled antibody KPEP11_18 against EphA4 N-terminal fragments in the cerebrospinal fluid.
Figure 14 shows the reactivity result of sandwich ELISA constructed employing antibody KPEP11_18 and HRP-labeled antibody KPEP11_10 against human EphA4 extracellular domain.
Figure 15 shows the reactivity result of sandwich ELISA constructed employing antibody KPEP11_18 and HRP-labeled antibody KPEP11_10 against EphA4 N-terminal fragments in the cerebrospinal fluid.
Figure 16 shows the reactivity result of sandwich ELISA constructed employing antibody KPEP11_10 and HRP-labeled antibody KPEP11_04 against human EphA4 extracellular domain.
Figure 17 shows the reactivity result of sandwich ELISA constructed employing antibody KPEP11_10 and HRP-labeled antibody KPEP11_04 against EphA4 N-terminal fragments in the cerebrospinal fluid.
Figure 18 shows the reactivity result of sandwich ELISA constructed employing antibody KPEP11_18 and HRP-labeled antibody KPEP11_08 against human EphA4 extracellular domain.
Figure 19 shows the reactivity result of sandwich ELISA constructed employing antibody KPEP11_18 and HRP-labeled antibody KPEP11_08 against EphA4 N-terminal fragments in the cerebrospinal fluid.
Figure 20 shows the result of evaluating reactivity of sandwich ELISA constructed employing antibody KPEP11_10 with HRP-labeled antibody KPEP11_04, HRP-labeled antibody KPEP11_18, and HRP-labeled EphA4 antibody (Sino or R&D) against human EphA4 extracellular domain.
Figure 21 shows the result of evaluating reactivity of sandwich ELISA constructed employing antibody KPEP11_10 with HRP-labeled antibody KPEP11_04, HRP-labeled antibody KPEP11_18, and HRP-labeled EphA4 antibody (Sino or R&D) against EphA4 N-terminal fragments in human plasma.
Figure 22 shows the result of evaluating reactivity of sandwich ELISA constructed employing antibody KPEP11_10 with HRP-labeled antibody KPEP11_04, HRP-labeled antibody KPEP11_18, and HRP-labeled EphA4 antibody (Sino or R&D) against EphA4 N-terminal fragments in human cerebrospinal fluid.
Figure 23 shows the result of evaluating reactivity of sandwich ELISA constructed employing antibody KPEP11_18 with HRP-labeled antibody KPEP11_08, HRP-labeled antibody KPEP11_10, and HRP-labeled EphA4 antibody (Sino or R&D) against human EphA4 extracellular domain.
Figure 24 shows the result of evaluating reactivity of sandwich ELISA constructed employing antibody KPEP11_18 with HRP-labeled antibody KPEP11_08, HRP-labeled antibody KPEP11_10, and HRP-labeled EphA4 antibody (Sino or R&D) against EphA4 N-terminal fragments in human plasma.
Figure 25 shows the result of evaluating reactivity of sandwich ELISA constructed employing antibody KPEP11_18 with HRP-labeled antibody KPEP11_08, HRP-labeled antibody KPEP11_10, and HRP-labeled EphA4 antibody (Sino or R&D) against EphA4 N-terminal fragments in human cerebrospinal fluid.
Figure 26 shows the correlation analysis result carried out based on the quantification result of EphA4 N-terminal fragments in human cerebrospinal fluid analyzed by LC-MS and the quantification result of EphA4 N-terminal fragments in human cerebrospinal fluid analyzed in Quantification analysis by ELISA analysis 1.
Figure 27 shows the correlation analysis result carried out based on the quantification result of EphA4 N-terminal fragments in human cerebrospinal fluid analyzed by LC-MS and the quantification result of EphA4 N-terminal fragments in human cerebrospinal fluid analyzed in Quantification analysis by ELISA analysis 2.

### Description of Embodiments

Regions specified or encoded by SEQ ID NOs. used herein are as follows:

| SEQ No | Sequence Region | SEQ No | Sequence Region |
|---|---|---|---|
| 1 | Human EphA4 full-length (amino acid sequence) | 32 | KPEP11_10 full-length heavy chain (amino acid sequence) |
| 2 | Extracellular domain of human EphA4 (amino acid sequence) | 33 | KPEP11_10 full-length light chain (amino acid sequence) |
| 3 | Human EphA4 extracellular domain-SEAP-His protein (amino acid sequence) | 34 | KPEP11_10 full-length heavy chain (nucleic acid sequence) |
| 4 | Human EphA4 extracellular domain-His protein (amino acid sequence) | 35 | KPEP11_10 full-length light chain (nucleic acid sequence) |
| 5 | Human EphA4 extracellular domain-MBP-His protein (amino acid sequence) | 36 | KPEP11_18 full-length heavy chain (amino acid sequence) |
| 6 | KPEP11_04 heavy chain variable region (amino acid sequence) | 37 | KPEP11_18 full-length light chain (amino acid sequence) |
| 7 | KPEP11_04 light chain variable region (amino acid sequence) | 38 | KPEP11_18 full-length heavy chain (nucleic acid sequence) |
| 8 | KPEP11_04 heavy chain variable region (nucleic acid sequence) | 39 | KPEP11_18 full-length light chain (nucleic acid sequence) |
| 9 | KPEP11_04 light chain variable region (nucleic acid sequence) | 40 | KPEP11_04 heavy chain CDR1 (amino acid sequence) |
| 10 | KPEP11_08 heavy chain variable region (amino acid sequence) | 41 | KPEP11_04 heavy chain CDR2 (amino acid sequence) |
| 11 | KPEP11_08 light chain variable region (amino acid sequence) | 42 | KPEP11_04 heavy chain CDR3 (amino acid sequence) |
| 12 | KPEP11_08 heavy chain variable region (nucleic acid sequence) | 43 | KPEP11_04 light chain CDR2 (amino acid sequence) |
| 13 | KPEP11_08 light chain variable region (nucleic acid sequence) | 44 | KPEP11_04 light chain CDR2 (amino acid sequence) |
| 14 | KPEP11_10 heavy chain variable region (amino acid sequence) | 45 | KPEP11_04 light chain CDR3 (amino acid sequence) |
| 15 | KPEP11_10 light chain variable region (amino acid sequence) | 46 | KPEP11_04 heavy chain CDR1 (nucleic acid sequence) |
| 16 | KPEP11_10 heavy chain variable region (nucleic acid sequence) | 47 | KPEP11_04 heavy chain CDR2 (nucleic acid sequence) |
| 17 | KPEP11_10 light chain variable region (nucleic acid sequence) | 48 | KPEP11_04 heavy chain CDR3 (nucleic acid sequence) |
| 18 | KPEP11_18 heavy chain variable region (amino acid sequence) | 49 | KPEP11_04 light chain CDR2 (nucleic acid sequence) |
| 19 | KPEP11_18 light chain variable region (amino acid sequence) | 50 | KPEP11_04 light chain CDR2 (nucleic acid sequence) |
| 20 | KPEP11_18 heavy chain variable region (nucleic acid sequence) | 51 | KPEP11_04 light chain CDR3 (nucleic acid sequence) |
| 21 | KPEP11_18 light chain variable region (nucleic acid sequence) | 52 | KPEP11_08, KPEP11_10 heavy chain CDR1 (amino acid sequence) |
| 22 | Constant region of rabbit IgG (amino acid sequence) | 53 | KPEP11_08, KPEP11_10 heavy chain CDR2 (amino acid sequence) |
| 23 | Constant region of rabbit Igκ (amino acid sequence) | 54 | KPEP11_08, KPEP11_10 heavy chain CDR3 (amino acid sequence) |
| 24 | KPEP11_04 full-length heavy chain (amino acid sequence) | 55 | KPEP11_08, KPEP11_10 light chain CDR1 (amino acid sequence) |
| 25 | KPEP11_04 full-length light chain (amino acid sequence) | 56 | KPEP11_08, KPEP11_10 light chain CDR2 (amino acid sequence) |
| 26 | KPEP11_04 full-length heavy chain (nucleic acid sequence) | 57 | KPEP11_08, KPEP11_10 light chain CDR3 (amino acid sequence) |
| 27 | KPEP11_04 full-length light chain (nucleic acid sequence) | 58 | KPEP11_08, KPEP11_10 heavy chain CDR1 (nucleic acid sequence) |
| 28 | KPEP11_08 full-length heavy chain (amino acid sequence) | 59 | KPEP11_08, KPEP11_10 heavy chain CDR2 (nucleic acid sequence) |
| 29 | KPEP11_08 full-length light chain (amino acid sequence) | 60 | KPEP11_08, KPEP11_10 heavy chain CDR3 (nucleic acid sequence) |
| 30 | KPEP11_08 full-length heavy chain (nucleic acid sequence) | 61 | KPEP11_08, KPEP11_10 light chain CDR1 (nucleic acid sequence) |
| 31 | KPEP11_08 full-length light chain (nucleic acid sequence) | 62 | KPEP11_08, KPEP11_10 light chain CDR2 (nucleic acid sequence) |
| 63 | KPEP11_08, KPEP11_10 light chain CDR3 (nucleic acid sequence) | 95 | Light chain CDR1 of KPEP11_01, KPEP11_09, KPEP11_12, and KPEP11_13 (amino acid sequence) |
| 64 | KPEP11_18 heavy chain CDR1 (amino acid sequence) | 96 | Light chain CDR2 of KPEP11_01, KPEP11_09, KPEP11_12, and KPEP11_13 (amino acid sequence) |
| 65 | KPEP11_18 heavy chain CDR2 (amino acid sequence) | 97 | Light chain CDR3 of KPEP11 01, KPEP11_09, KPEP11_12, and KPEP11_13 (amino acid sequence) |
| 66 | KPEP11_18 heavy chain CDR3 (amino acid sequence) | 98 | Heavy chain CDR1 of KPEP11_02, KPEP11_05, and KPEP11_07 (amino acid sequence) |
| 67 | KPEP11_18 light chain CDR1 (amino acid sequence) | 99 | Heavy chain CDR2 of KPEP11_02 and KPEP11_05 (amino acid sequence) |
| 68 | KPEP11_18 light chain CDR2 (amino acid sequence) | 100 | Heavy chain CDR3 of KPEP11_02 and KPEP11_05 (amino acid sequence) |
| 69 | KPEP11_18 light chain CDR3 (amino acid sequence) | 101 | Light chain CDR1 of KPEP11_02, KPEP11_05, and KPEP11_07 (amino acid sequence) |
| 70 | KPEP11_18 heavy chain CDR1 (nucleic acid sequence) | 102 | Light chain CDR2 of KPEP11_02, KPEP11_05, and KPEP11_07 (amino acid sequence) |
| 71 | KPEP11_18 heavy chain CDR2 (nucleic acid sequence) | 103 | Light chain CDR3 of KPEP11_02, KPEP11_05, and KPEP11_07 (amino acid sequence) |
| 72 | KPEP11_18 heavy chain CDR3 (nucleic acid sequence) | 104 | Heavy chain CDR2 of KPEP11_07 (amino acid sequence) |
| 73 | KPEP11_18 light chain CDR1 (nucleic acid sequence) | 105 | Heavy chain CDR3 of KPEP11_07 (amino acid sequence) |
| 74 | KPEP11_18 light chain CDR2 (nucleic acid sequence) | 106 | Heavy chain CDR2 of KPEP11_12, KPEP11_13 (amino acid sequence) |
| 75 | KPEP11_18 light chain CDR3 (nucleic acid sequence) | 107 | Heavy chain CDR1 of KPEP11_20 (amino acid sequence) |
| 76 | Heavy chain variable region of KPEP11_01 (amino acid sequence) | 108 | Heavy chain CDR2 of KPEP11_20 (amino acid sequence) |
| 77 | Light chain variable region of KPEP11_01 (amino acid sequence) | 109 | Heavy chain CDR3 of KPEP11_20 (amino acid sequence) |
| 78 | Heavy chain variable region of KPEP11_02 (amino acid sequence) | 110 | Light chain CDR1 of KPEP11_20 (amino acid sequence) |
| 79 | Light chain variable region of KPEP11_02 (amino acid sequence) | 111 | Light chain CDR2 of KPEP11_20 (amino acid sequence) |
| 80 | Heavy chain variable region of KPEP11_05 (amino acid sequence) | 112 | Light chain CDR3 of KPEP11_20 (amino acid sequence) |
| 81 | Light chain variable region of KPEP11_05 (amino acid sequence) | 113 | Monkey EphA4 full-length (amino acid sequence) |
| 82 | Heavy chain variable region of KPEP11_07 (amino acid sequence) | 114 | Monkey EphA4 extracellular domain (amino acid sequence) |
| 83 | Light chain variable region of KPEP11_07 (amino acid sequence) | 115 | Rabbit EphA4 full-length (amino acid sequence) |
| 84 | Heavy chain variable region of KPEP11_09 (amino acid sequence) | 116 | Rabbit EphA4 extracellular domain (amino acid sequence) |
| 85 | Light chain variable region of KPEP11_09 (amino acid sequence) | 117 | Rat EphA4 full-length (amino acid sequence) |
| 86 | Heavy chain variable region of KPEP11_12 (amino acid sequence) | 118 | Rat EphA4 extracellular domain (amino acid sequence) |
| 87 | Light chain variable region of KPEP11_12 (amino acid sequence) | 119 | Mouse EphA4 full-length (amino acid sequence) |
| 88 | Heavy chain variable region of KPEP11_13 (amino acid sequence) | 120 | Mouse EphA4 extracellular domain (amino acid sequence) |
| 89 | Light chain variable region of KPEP11_13 (amino acid sequence) | 121 | Human EphA4 signal sequence (amino acid sequence) |
| 90 | Heavy chain variable region of KPEP11_20 (amino acid sequence) | 122 | Signal sequence of preprotrypsin (amino acid sequence) |
| 91 | Light chain variable region of KPEP11_20 (amino acid sequence) | 123 | Ligand binding domain of human EphA4 (amino acid sequence) |
| 92 | Heavy chain CDR1 of KPEP11_01, KPEP11_09, KPEP11_12, KPEP11_13 (amino acid sequence) | 124 | Fibronectin III-type domain 1 of human EphA4 (amino acid sequence) |
| 93 | Heavy chain CDR2 of KPEP11 01, KPEP11_09 (amino acid sequence) | 125 | Fibronectin III-type domain 2 of human EphA4 |
| 94 | Heavy chain CDR3 of KPEP11_01, KPEP11_09, KPEP11_12, and KPEP11_13 (amino acid sequence) | 126 | MBP-His protein (amino acid sequence) |

The present disclosure relates to an anti-EphA4 antibody that binds to EphA4.

The anti-EphA4 antibody according to the present disclosure is an antibody that can specifically recognize and bind to EphA4. The anti-EphA4 antibody may be an intact antibody, or may be a synthetic antibody (such as a recombinant antibody, a chimeric antibody, and a humanized antibody), as long as it has binding affinity with EphA4. EphA4 can be recognized herein to refer to human, mouse, rat, rabbit, or monkey-derived EphA4. Human, mouse, rat, rabbit, and monkey-derived EphA4 can be obtained from public database in which sequence information in registered, such as Genbank provided by the U.S. National Center for Biotechnology Information, or sequence information of EphA4 gene can be obtained by designing primers based on the EphA4 base sequence information of closely related animal species, and then cloning from RNA extracted from the desired animal species. For example, the base sequence information of human, mouse, rat, rabbit, and monkey EphA4 is registered in the database respectively as Genbank Accession No. NM_004438.5, NM_007936.3, NM_001162411.1, XM_002712496.3, and NM_001260870.1.

In one aspect of the present disclosure, EphA4 comprises the amino acid sequence shown in SEQ ID NO. 1 or an amino acid sequence having one or more amino acids substituted/added/deleted in said amino acid sequence. "Multiple" herein is not limited as long as it retains a functional property equivalent to its original sequence, and is from 2 to 100, such as from 2 to 90, from 2 to 80, from 2 to 70, from 2 to 60, from 2 to 50, from 2 to 40, from 2 to 30, from 2 to 20, from 2 to 10, 9, 8, 7, 6, 5, 4, 3, or 2, or 10% or less, such as 9% or less, 8% or less, 7% or less, 6% or less, 5% or less, 4% or less, 3% or less, 2% or less, or 1% or less of the number of amino acids of the amino acid sequence.

In the present disclosure, the term "specific binding" is a term well-known to those skilled in the art in the technical field, and methods for determining specific binding of antibodies or antigen binding fragments thereof against antigens or epitopes are also well-known. In one embodiment, "specific binding" is understood as that the anti-EphA4 antibody or antigen binding fragment thereof can bind to EphA4 by immunological reaction with a larger binding affinity and binding activity, and more rapidly and/or for a longer period of time than to other target molecules. In another embodiment, "specific binding" may be shown by an antibody having at least about 10⁻⁷ M, or at least about 10⁻⁸ M, or at least about 10⁻⁹ M, or at least 10⁻¹⁰ M, or less KD against EphA4. Also, in further another embodiment, "specific binding" is understood as binding to EphA4 by immunological reaction but essentially not binding to other family molecules of the Eph receptor (such as EphA1, EphA2, EphA3, EphA5, EphA6, EphA7, EphA8, EphA10, EphB1, EphB2, EphB3, EphB4, and EphB6) .

An "antigen binding fragment" is not particularly limited as long as it is a fragment of an anti-EphA4 antibody that maintains specific binding against EphA4, and includes e.g. Fab, Fab', F(ab')₂, Fv, and scFv.

Methods well-known to those skilled in the art in the technical field may be employed as the method for measuring the binding property to an antigen (such as binding affinity and cross-species reactivity) of an anti-EphA4 antibody or an antigen binding fragment thereof. For example, binding affinity may be measured using, but is not limited to, Biacore^{™} biosensor, KinExA biosensor, scintillation proximity assay, ELISA, ORIGEN immunoassay (from IGEN), flow cytometry, fluorescence quenching, fluorescence metastasis, yeast display, and/or immunostaining.

The anti-EphA4 antibody or antigen binding fragment thereof according to the present disclosure may be of any class such as IgG, IgA, or IgM (or subclasses thereof), and is not limited to a particular class. Immunoglobulins are classified into different classes depending on the antibody amino acid sequence of the constant region of the heavy chain (sometimes referred to as the H-chain). There are five major immunoglobulin classes: IgA, IgD, IgE, IgG, and IgM, several of which may be further subdivided into subclasses (isotypes) such as IgG₁, IgG₂, IgG₃, IgG₄, and IgA₁ and IgA₂. the constant regions of the heavy chains corresponding to the different classes of immunoglobulins are referred to as α, δ, ε, γ, and µ, respectively. Moreover, as for the types of the light chain of an antibody (sometimes referred to as the L-chain), there are λ and κ chains.

The anti-EphA4 antibody or antigen binding fragment thereof according to the present disclosure may be an IgG antibody. The anti-EphA4 antibody according to the present disclosure may also be in some cases in the form of a monomer, dimer, or a multimer.

The variable region of an antibody or an antigen binding fragment thereof according to the present disclosure may mean the variable region of an antibody light chain and/or the variable region of an antibody heavy chain, and the constant region of an antibody may mean the constant region of an antibody light chain and/or the constant region of an antibody heavy chain. The variable region of the heavy chain and light chain each consist of four framework regions (FR) connected by three CDRs also known as complementarity determining regions. The CDRs in each chain are retained in vicinity by FRs, and together with the CDRs in the other chain, contribute to the formation of the antigen binding site of the antibody. Technologies for determining the CDR include, but are not limited to, e.g. (1) an approach based on cross-species sequence variability (such as Kabat et al, Sequences of Proteins of Immunological Interest, 5th ed., 1991, National Institutes of Health, Bethesda MD) ; and (2) an approach based on the crystal structure research of the antigen-antibody complex (Al-lazikani et al., 1997 J. Molec. Biol. 273:927-948). These and other approaches may be employed in combination.

In the anti-EphA4 antibody or antigen binding fragment thereof according to the present disclosure, for example, without being limited thereto, human, mouse, rat, rabbit, or monkey-derived heavy chain sequence and/or light chain sequence can be employed. In one embodiment, the anti-EphA4 antibody or antigen binding fragment thereof according to the present disclosure has rabbit-derived heavy chain sequence and light chain sequence.

The anti-EphA4 antibody or antigen binding fragment thereof according to the present disclosure may be modified as desired. Modification of the anti-EphA4 antibody or antigen binding fragment thereof may be modifications that change (a) the three-dimensional structure of the amino acid sequence in the modification region such as sheet or helix conformation; (b) the charge or hydrophobic condition of the molecule at the target site; or (c) the effect of modification against maintenance of the side chain volume, or may be modifications in which these changes are not apparently observed.

The modification of the anti-EphA4 antibody or antigen binding fragment thereof according to the present disclosure may be achieved by e.g. substitutions, deletions, and additions of the configuring amino acid residues.

In the present specification, an amino acid is employed in its broadest meaning, and includes not only natural amino acids, such as serine (Ser), asparagine (Asn), valine (Val), leucine (Leu), isoleucine (Ile), alanine (Ala), tyrosine (Tyr), glycine (Gly), lysine (Lys), arginine (Arg), histidine (His), aspartic acid (Asp), glutamic acid (Glu), glutamine (Gin), threonine (Thr), cysteine (Cys), methionine (Met), phenylalanine (Phe), tryptophan (Trp), and proline (Pro), but also non-natural amino acids such as amino acid variants and derivatives. Those skilled in the art, in consideration of this broad definition, naturally recognize that amino acids herein include, for example, L-amino acids; D-amino acids; chemically modified amino acids such as amino acid variants and amino acid derivatives; amino acids that are not components of proteins in vivo such a norleucine, β-alanine, and ornithine; and chemically synthesized compounds having amino acid properties well-known to those skilled in the art. Examples of non-natural amino acids include, e.g., α-methylamino acids (such as α-methylalanine), D-amino acids (such as D-aspartic acid and D-glutamic acid), histidine-like amino acids (such as 2-amino-histidine, β-hydroxy-histidine, homohistidine, α-fluoromethyl-histidine, and α-methyl-histidine), amino acids having excess methylenes on the side chain ("homo" amino acids), and amino acids having the carboxylate functional group amino acid in the side chain substituted with a sulfonate group (such as cysteic acid).

For example, naturally-occurring amino acid residues may be classified into the following groups based on general side chain properties:
(1) Hydrophobic: Met, Ala, Val, Leu, and Ile;
(2) Neutral hydrophilic: Asn, Gln, Cys, Ser, and Thr;
(3) Acidic: Asp and Glu;
(4) Basic: His, Lys, and Arg;
(5) Residues that influence chain orientation: Gly and Pro; and
(6) Aromatic: Trp, Tyr, and Phe.

Nonconservative substitutions of the amino acid sequence configuring an antibody may be carried out by exchanging an amino acid belonging to one of these groups with an amino acid belonging to another group. A more conservative substitution may be carried out by exchanging an amino acid belonging to one of these groups with another amino acid in the same group. Similarly, deletion or substitution of an amino acid sequence may be appropriately carried out.

Examples of modifications of the amino acid configuring an antibody may be for example sugar glycosylation and post-translational modification such as acetylation or phosphorylation. An antibody may be glycosylated at a conserved position in its constant region. Glycosylation of an antibody is ordinarily either N-linked or O-linked. N-linked means the binding of a carbohydrate portion on the side chain of an asparagine residue. Tripeptide sequences asparagine-X-serine, asparagine-X-threonine, and asparagine-X-cysteine (wherein X is any amino acid other than proline) are recognition sequences for enzymatically adding a carbohydrate portion on an asparagine side chain. A potential glycosylation site exists when any of these tripeptide sequences is present in the antibody. O-linked glycosylation may be the binding of any of N-acetylgalactosamine, galactose, or xylose to a hydroxy amino acid (such as serine or threonine), and in some cases, to 5-hydroxy proline or 5-hydroxy lysine. Those skilled in the art can appropriately select the glycosylation condition according to the purpose (such as the type of host cell or cell medium, pH, etc., when glycosylation is performed with a biological method).

The anti-EphA4 antibody or antigen binding fragment thereof according to the present disclosure may be further modified by other modification methods alone or in combination based on the technical common sense well-known to those skilled in the art.

The anti-EphA4 antibody or antigen binding fragment thereof according to the present disclosure can be produced by a method well-known to those skilled in the art. For example, the antibody or antigen binding fragment thereof may be produced by integrating a nucleic acid encoding the anti-EphA4 antibody or antigen binding fragment thereof according to the present disclosure into an expression vector, introducing the expression vector into a host cell, and culturing the host cell. Accordingly, the present disclosure encompasses a nucleic acid encoding the anti-EphA4 antibody or antigen binding fragment thereof, a vector comprising the nucleic acid, a host cell comprising the vector, as well as a method for producing an anti-EphA4 antibody or an antigen binding fragment thereof comprising a step of culturing the host cell.

The nucleic acid encoding the anti-EphA4 antibody or antigen binding fragment thereof according to the present disclosure may possess a DNA encoding the signal sequence, and may possess a DNA encoding the signal sequence on the 5' terminal of the DNA encoding the heavy chain variable region and the DNA encoding the light chain variable region. A signal sequence is an amino acid residue present on the N-terminal of the protein, which is necessary for the secretory protein or the integral membrane protein to pass through the lipid bilayer after being synthesized on ribosomes, and in the present disclosure, is not particularly limited as long as it is a sequence having this function. Signal sequences which the anti-EphA4 antibody or antigen binding fragment thereof according to the present disclosure may comprise include signal sequences derived from human, mouse, rat, rabbit, donkey, goat, horse, bird, dog, cat, yeast, and the like.

The anti-EphA4 antibody or antigen binding fragment thereof according to the present disclosure may be those that are isolated or purified according to methods well-known to those skilled in the art.

In the present specification, "isolated" or "purified" means that it is artificially isolated or purified from the natural state. When a molecule or a composition is naturally occurring, if is it changed or removed from its original environment, or both, it is "isolated" or "purified". Examples of isolation or purification methods include, but are not limited to, e.g., electrophoresis, molecular biological, immunological, or chromatography methods, specifically, ion exchange chromatography, hydrophobic chromatography, reverse phase HPLC chromatography, isoelectric focusing, or alkali extraction methods.

In one aspect, the anti-EphA4 antibody or antigen binding fragment thereof according to the present disclosure comprises the following CDRs:
a heavy chain comprising a heavy chain CDR1 consisting of the amino acid sequence shown in SEQ ID NO. 40; a heavy chain CDR2 consisting of the amino acid sequence shown in SEQ ID NO. 41; and a heavy chain CDR3 consisting of the amino acid sequence shown in SEQ ID NO. 42; and
a light chain comprising a light chain CDR1 consisting of the amino acid sequence shown in SEQ ID NO. 43; a light chain CDR2 consisting of the amino acid sequence shown in SEQ ID NO. 44; and a light chain CDR3 consisting of the amino acid sequence shown in SEQ ID NO. 45.

In another aspect, the anti-EphA4 antibody or antigen binding fragment thereof according to the present disclosure comprises the following CDRs:
a heavy chain comprising a heavy chain CDR1 consisting of the amino acid sequence shown in SEQ ID NO. 52; a heavy chain CDR2 consisting of the amino acid sequence shown in SEQ ID NO. 53; and a heavy chain CDR3 consisting of the amino acid sequence shown in SEQ ID NO. 54; and
a light chain comprising a light chain CDR1 consisting of the amino acid sequence shown in SEQ ID NO. 55; a light chain CDR2 consisting of the amino acid sequence shown in SEQ ID NO. 56; and a light chain CDR3 consisting of the amino acid sequence shown in SEQ ID NO. 57;

In further another aspect, the anti-EphA4 antibody or antigen binding fragment thereof according to the present disclosure comprises the following CDRs:
a heavy chain comprising a heavy chain CDR1 consisting of the amino acid sequence shown in SEQ ID NO. 64; a heavy chain CDR2 consisting of the amino acid sequence shown in SEQ ID NO. 65; and a heavy chain CDR3 consisting of the amino acid sequence shown in SEQ ID NO. 66; and
a light chain comprising a light chain CDR1 consisting of the amino acid sequence shown in SEQ ID NO. 67; a light chain CDR2 consisting of the amino acid sequence shown in SEQ ID NO. 68; and a light chain CDR3 consisting of the amino acid sequence shown in SEQ ID NO. 69.

In one embodiment, the anti-EphA4 antibody or antigen binding fragment thereof comprises a heavy chain variable region consisting of the amino acid sequence shown in SEQ ID NO. 6 and a light chain variable region consisting of the amino acid sequence shown in SEQ ID NO. 7.

In one embodiment, the anti-EphA4 antibody or antigen binding fragment thereof comprises a heavy chain variable region consisting of the amino acid sequence shown in SEQ ID NO. 10 and a light chain variable region consisting of the amino acid sequence shown in SEQ ID NO. 11.

In another embodiment, the anti-EphA4 antibody or antigen binding fragment thereof comprises a heavy chain variable region consisting of the amino acid sequence shown in SEQ ID NO. 14 and a light chain variable region consisting of the amino acid sequence shown in SEQ ID NO. 15.

In further another embodiment, the anti-EphA4 antibody or antigen binding fragment thereof comprises a heavy chain variable region consisting of the amino acid sequence shown in SEQ ID NO. 18 and a light chain variable region consisting of the amino acid sequence shown in SEQ ID NO. 19.

In the present disclosure, the variable region of the heavy chain and/or the variable region of the light chain may have an amino acid sequence in which one or more amino acids are substituted, added, and/or deleted to/from the original sequence. "Multiple" herein is not limited as long as it retains binding affinity against EphA4 and promotes cleaving of EphA4, and is from 2 to 15 or from 2 to 10, such as 9, 8, 7, 6, 5, 4, 3, or 2, or 10% or less, such as 9% or less, 8% or less, 7% or less, 6% or less, 5% or less, 4% or less, 3% or less, 2% or less, or 1% or less of the number of amino acids of the amino acid sequence.

In one embodiment, the heavy chain of the anti-EphA4 antibody or antigen binding fragment thereof according to the present disclosure comprises the constant region of rabbit IgG. In a particular embodiment, the constant region of rabbit IgG comprises the amino acid sequence of SEQ ID NO. 22.

In one embodiment, the light chain of the anti-EphA4 antibody or antigen binding fragment thereof according to the present disclosure comprises the constant region of rabbit Igκ. In a particular embodiment, the constant region of rabbit Igκ comprises the amino acid sequence of SEQ ID NO. 23.

In one embodiment, the heavy chain of the anti-EphA4 antibody according to the present disclosure comprises the amino acid sequence shown in SEQ ID NO. 24 and the light chain of the anti-EphA4 antibody comprises the amino acid sequence shown in SEQ ID NO. 25.

In one embodiment, the heavy chain of the anti-EphA4 antibody according to the present disclosure comprises the amino acid sequence shown in SEQ ID NO. 28 and the light chain of the anti-EphA4 antibody comprises the amino acid sequence shown in SEQ ID NO. 29.

In one embodiment, the heavy chain of the anti-EphA4 antibody according to the present disclosure comprises the amino acid sequence shown in SEQ ID NO. 32 and the light chain of the anti-EphA4 antibody comprises the amino acid sequence shown in SEQ ID NO. 33.

In one embodiment, the heavy chain of the anti-EphA4 antibody according to the present disclosure comprises the amino acid sequence shown in SEQ ID NO. 36 and the light chain of the anti-EphA4 antibody comprises the amino acid sequence shown in SEQ ID NO. 37.

In one embodiment, the anti-EphA4 antibody or antigen binding fragment thereof according to the present disclosure is labeled. In the present disclosure, "label" means a detectable compound or composition that is directly or indirectly bound to the antibody or antigen binding fragment thereof. The label may be detectable per se, or may be detectable by a combination with another specific binding pair, and for example, in the case of an enzyme label, a detectable signal may be produced by acting on or reacting with a substrate compound or composition.

In another embodiment, for example, for reasons such as reducing the inhomogeneity of antibodies produced by antibody-producing cell (U.S. Patent Application Publication No. 2010/0297697 or Liu H et al., MAbs. 2014 Sep-Oct; 6(5): 1145-1154), the anti-EphA4 antibody may have lysine positioned at the C-terminal (carboxy terminal) of the heavy chain deleted. In the present disclosure, an anti-EphA4 antibody having the C-terminal lysine of the heavy chain deleted includes an anti-EphA4 antibody having the C-terminal lysine of the heavy chain deleted by genetic modification, or an anti-EphA4 antibody having the C-terminal lysine of the heavy chain post-translationally cleaved by a carboxypeptidase etc., and the like. Moreover, in the present disclosure, an anti-EphA4 antibody having the C-terminal lysine of the heavy chain deleted includes not only an anti-EphA4 antibody having the C-terminal lysine deleted in both heavy chains, but also an anti-EphA4 antibody having C-terminal lysine deleted in only one of the heavy chains.

In one aspect, the present disclosure relates to an isolated nucleic acid encoding an anti-EphA4 antibody or an antigen binding fragment thereof. The isolated nucleic acid encoding an anti-EphA4 antibody or an antigen binding fragment thereof refers to one or more nucleic acid molecules encoding the heavy chain and/or light chain of an anti-EphA4 antibody or an antigen binding fragment thereof. In one embodiment, the nucleic acid according to the present disclosure encodes the heavy chain of an anti-EphA4 antibody or an antigen binding fragment thereof. In another embodiment, the nucleic acid according to the present disclosure encodes the light chain of an anti-EphA4 antibody or an antigen binding fragment thereof. In further another embodiment, the nucleic acid according to the present disclosure encodes the heavy chain and light chain of an anti-EphA4 antibody or an antigen binding fragment thereof. The nucleic acid according to the present disclosure includes a first nucleic acid molecule encoding the heavy chain an anti-EphA4 antibody or an antigen binding fragment thereof and a second nucleic acid molecule encoding the light chain of an anti-EphA4 antibody or an antigen binding fragment thereof.

In another aspect, the present disclosure relates to a vector comprising an isolated nucleic acid encoding an anti-EphA4 antibody or an antigen binding fragment thereof. The vector according to the present disclosure refers to one or more vectors comprising an isolated nucleic acid encoding an anti-EphA4 antibody or an antigen binding fragment thereof. In one embodiment, the vector according to the present disclosure is a vector comprising the nucleic acid encoding the heavy chain of an anti-EphA4 antibody or an antigen binding fragment thereof and the nucleic acid encoding the light chain of an anti-EphA4 antibody or an antigen binding fragment thereof. In another embodiment, the vector according to the present disclosure is a vector comprising the nucleic acid encoding the heavy chain and light chain of an anti-EphA4 antibody or an antigen binding fragment thereof. In further another embodiment, the vector according to the present disclosure comprises a first vector comprising the nucleic acid encoding the heavy chain of an anti-EphA4 antibody or an antigen binding fragment thereof and a second vector comprising the nucleic acid encoding the light chain of an anti-EphA4 antibody or an antigen binding fragment thereof. The vector according to the present disclosure may be, but is not particularly limited thereto, a plasmid, a cosmid, a virus, a phage, and the like. For example, as a viral vector, retrovirus, lentivirus, adenovirus, adeno-associated virus, or herpes simplex virus vector, and the like are also included in the vector according to the present disclosure.

In further another aspect, a host cell comprising the vector according to the present disclosure, as well as a method for producing an anti-EphA4 antibody or an antigen binding fragment thereof comprising a step of culturing the host cell are also included in the present disclosure. The host cell according to the present disclosure may be, but is not particularly limited thereto, an E. coli cell, a monkey COS cell, a Chinese hamster ovary (CHO) cell, a NS0 cell, and the like. In one embodiment, the method for producing an anti-EphA4 antibody or an antigen binding fragment thereof comprises a step of culturing a host cell, and a step of collecting the anti-EphA4 antibody or an antigen binding fragment thereof secreted from the host cell (or culture medium of the host cell).

The anti-EphA4 antibody or antigen binding fragment thereof according to the present disclosure characterized by the above CDRs binds to any region of the N-terminal of EphA4. In the present disclosure, the "N-terminal domain" of EphA4 refers to the extracellular domain (ECD) of EphA4, or the N-terminal side region when EphA4 is cleaved by matrix metalloprotease (MMP) or ADAM (a disintegrin and metalloproteinase). Note that the ECD in a human EphA4 is defined as those having the amino acid sequence shown in SEQ ID NO. 2, or an amino acid sequence in which one or more amino acids are substituted, added, and/or deleted in the amino acid sequence. "Multiple" herein is from 2 to 15 or from 2 to 10, such as 9, 8, 7, 6, 5, 4, 3, or 2, or 10% or less, such as 9% or less, 8% or less, 7% or less, 6% or less, 5% or less, 4% or less, 3% or less, 2% or less, or 1% or less of the number of amino acids of the amino acid sequence.

The anti-EphA4 antibody or antigen binding fragment thereof according to the present disclosure can specifically bind to EphA4 and detect EphA4 with high detection sensitivity. Accordingly, in another aspect, the present disclosure relates to a method for detecting or quantifying EphA4 in a biological sample employing the anti-EphA4 antibody or antigen binding fragment thereof according to the present disclosure (hereinafter also referred to as the method according to the present disclosure).

In the method according to the present disclosure, the "EphA4" to be the measurement target includes full-length EphA4, as well as a fragment consisting of the N-terminal domain of EphA4 (herein also referred to simply as "EphA4 N-terminal fragment" or "N-terminal fragment of EphA4").

In the method according to the present disclosure, the biological sample is not particularly limited as long as it is a sample that may comprise full-length EphA4 or EphA4 N-terminal fragment, and e.g. can include biologically derived liquid components (also referred to as body fluid) such as blood, serum, plasma, cerebrospinal fluid (CSF), urine, saliva, lacrimal fluid, sweat, and the like. In one embodiment, the biological sample is blood, serum, plasma, or cerebrospinal fluid. Moreover, the biological sample is not limited to human-derived biological samples, and includes biological samples of animals other than human. Such animals can include, but are not limited to, e.g., mouse, rat, rabbit, monkey, and the like.

The method according to the present disclosure comprises contacting a biological sample with the anti-EphA4 antibody or antigen binding fragment thereof according to the present disclosure. Detection or quantification of EphA4 can be carried out with immunoassays well-known in the technical field.

The method according to the present disclosure can comprise contacting the biological sample and the anti-EphA4 antibody or antigen binding fragment thereof according to the present disclosure (first antibody), and then further contacting the biological sample with labeled anti-EphA4 antibody or antigen binding fragment thereof (second antibody). In the method, different antibodies are employed as the first antibody and the labeled second antibody.

Immunoassay employs a detectably labeled anti-EphA4 antibody or antigen binding fragment thereof or a detectably labeled anti-EphA4 antibody or antigen binding fragment thereof (secondary antibody). Depending on the antibody labeling method, these are classified into enzyme immunoassay (EIA or ELISA), radioimmunoassay (RIA), fluorescence immunoassay (FIA), fluorescence polarization immunoassay (FPIA), chemiluminescence immunoassay (CLIA), electrochemiluminescence immunoassay (ECLIA), and the like, any of which can be employed for the method according to the present disclosure.

For ELISA method, enzymes such as peroxidase and alkaline phosphatase can be employed, for RIA method, radioactive materials such as ¹²⁵I, ¹³¹I, ³⁵S, and ³H can be employed, for FPIA method, fluorescent substances such as fluorescein isothiocyanate, rhodamine, dansyl chloride, phycoerythrin, tetramethylrhodamine isothiocyanate, and near-infrared fluorescent material can be employed, and for CLIA method, enzymes such as luciferase and β galactosidase with luminescence substrates that changes to luminescent substances with each enzyme, as well as antibodies labeled with luminescent substances such as luciferin and aequorin can be employed. In addition, antibodies labeled with nanoparticles such as gold colloid and quantum dot can also be detected.

Also, in an immunoassay, an anti-EphA4 antibody or an antigen binding fragment thereof is labeled with biotin, bound to avidin or streptavidin labeled with an enzyme etc., and then EphA4 can be detected and measured.

In the ELISA method, for example the sandwich method can be employed. An anti-EphA4 antibody or an antigen binding fragment thereof is immobilized onto a solid phase support, an appropriately treated biological sample is added and allowed to react, and then another anti-EphA4 antibody or an antigen binding fragment thereof labeled with an enzyme is further added and allowed to react. After washing, this is reacted with the enzyme substrate, allowed to develop color, and the absorbance is measured to enable quantification of EphA4 or the N-terminal fragment of EphA4.

For the enzyme substrate, 3,3'-diaminobenzidne (DAB), 3,3',5,5'-tetramethylbenzidine (TMB or TMBZ), o-phenylenediamine (OPD), and the like can be employed when the enzyme is peroxidase, and p-nitrophenyl phosphate (NPP) and the like can be employed when the enzyme is alkaline phosphatase.

Moreover, among the above immunoassays, methods for enabling easy detection of trace amounts of protein include the aggregation method. Aggregation methods include e.g. latex aggregation method where a latex particle is bound to the antibody.

When a latex particle is bound to an anti-EphA4 antibody or an antigen binding fragment thereof and mixed with a biological sample, if EphA4 exists, the antibody-bound latex particles aggregate. Then, near-infrared light is irradiated to the sample, and aggregates are quantified by measurement of absorbance (turbidimetry) or measurement of scattered light (nephelometry) to determine antigen concentration.

In one embodiment, the method according to the present disclosure is a method for detecting or quantifying human EphA4 in a biological sample.

In one embodiment, the method according to the present disclosure a method for detecting or quantifying the N-terminal fragment of human EphA4 in a biological sample.

In one embodiment, the method according to the present disclosure employs sandwich ELISA which employs a combination of the antibody or antigen binding fragment thereof according to the present disclosure for detection or quantification of human EphA4.

In another aspect, the present disclosure also relates to a kit comprising the anti-EphA4 antibody or antigen binding fragment thereof according to the present disclosure for detecting or quantifying EphA4 (hereinafter also referred to as the kit according to the present disclosure). The kit according to the present disclosure can encompass any reagent or appliance which may be employed when detecting or quantifying EphA4, or instructions for using the kit.

In the kit according to the present disclosure, the "EphA4" that is the measurement target includes full-length EphA4, as well as the N-terminal fragment of EphA4.

In one embodiment, the kit according to the present disclosure relates to a kit for detecting or quantifying human EphA4.

In one embodiment, the kit according to the present disclosure relates to a kit for detecting or quantifying the N-terminal fragment of human EphA4.

In one embodiment, the kit according to the present disclosure comprises human full-length EphA4 that can be used as a positive control when creating the standard curve for human EphA4 or serial dilution solution of the full-length EphA4.

In one embodiment, the kit according to the present disclosure comprises the N-terminal fragment of human EphA4 that can be used as a positive control when creating the standard curve for human EphA4 or serial dilution solution of the fragment.

In one embodiment, the kit according to the present disclosure comprises an anti-EphA4 antibody or an antigen binding fragment thereof comprising a heavy chain variable region consisting of the amino acid sequence shown in SEQ ID NO. 14 and a light chain variable region consisting of the amino acid sequence shown in SEQ ID NO. 15; as well as an anti-EphA4 antibody or an antigen binding fragment thereof comprising a heavy chain variable region consisting of the amino acid sequence shown in SEQ ID NO. 18 and a light chain variable region consisting of the amino acid sequence shown in SEQ ID NO. 19.

In another embodiment, the kit according to the present disclosure comprises an anti-EphA4 antibody or an antigen binding fragment thereof comprising a heavy chain variable region consisting of the amino acid sequence shown in SEQ ID NO. 6 and a light chain variable region consisting of the amino acid sequence shown in SEQ ID NO. 7; as well as an anti-EphA4 antibody or an antigen binding fragment thereof comprising a heavy chain variable region consisting of the amino acid sequence shown in SEQ ID NO. 14 and a light chain variable region consisting of the amino acid sequence shown in SEQ ID NO. 15.

In another embodiment, the kit according to the present disclosure comprises an anti-EphA4 antibody or an antigen binding fragment thereof comprising a heavy chain variable region consisting of the amino acid sequence shown in SEQ ID NO. 10 and a light chain variable region consisting of the amino acid sequence shown in SEQ ID NO. 11; as well as an anti-EphA4 antibody or an antigen binding fragment thereof comprising a heavy chain variable region consisting of the amino acid sequence shown in SEQ ID NO. 18 and a light chain variable region consisting of the amino acid sequence shown in SEQ ID NO. 19.

In further another embodiment, the kit according to the present disclosure comprises an anti-EphA4 antibody or an antigen binding fragment thereof comprising a heavy chain variable region consisting of the amino acid sequence shown in SEQ ID NO. 6 and a light chain variable region consisting of the amino acid sequence shown in SEQ ID NO. 7; as well as an anti-EphA4 antibody or an antigen binding fragment thereof comprising a heavy chain variable region consisting of the amino acid sequence shown in SEQ ID NO. 10 and a light chain variable region consisting of the amino acid sequence shown in SEQ ID NO. 11.

The following can be exemplified as specific combinations of the anti-EphA4 antibody or antigen binding fragment thereof used in the method according to the present disclosure, in particular sandwich ELISA, or encompassed in the kit according to the present disclosure:

### (Solid Phase Antibody)

An antibody or an antigen binding fragment thereof comprising a heavy chain variable region consisting of the amino acid sequence shown in SEQ ID NO. 10 and a light chain variable region consisting of the amino acid sequence shown in SEQ ID NO. 11.

### (Labeled Antibody)

An antibody or an antigen binding fragment thereof comprising a heavy chain variable region consisting of the amino acid sequence shown in SEQ ID NO. 6 and a light chain variable region consisting of the amino acid sequence shown in SEQ ID NO. 7.

### (Solid Phase Antibody)

An antibody or an antigen binding fragment thereof comprising a heavy chain variable region consisting of the amino acid sequence shown in SEQ ID NO. 14 and a light chain variable region consisting of the amino acid sequence shown in SEQ ID NO. 15.

### (Labeled Antibody)

An antibody or an antigen binding fragment thereof comprising a heavy chain variable region consisting of the amino acid sequence shown in SEQ ID NO. 6 and a light chain variable region consisting of the amino acid sequence shown in SEQ ID NO. 7.

### (Solid Phase Antibody)

An antibody or an antigen binding fragment thereof comprising a heavy chain variable region consisting of the amino acid sequence shown in SEQ ID NO. 6 and a light chain variable region consisting of the amino acid sequence shown in SEQ ID NO. 7.

### (Labeled Antibody)

An antibody or an antigen binding fragment thereof comprising a heavy chain variable region consisting of the amino acid sequence shown in SEQ ID NO. 10 and a light chain variable region consisting of the amino acid sequence shown in SEQ ID NO. 11.

### (Solid Phase Antibody)

An antibody or an antigen binding fragment thereof comprising a heavy chain variable region consisting of the amino acid sequence shown in SEQ ID NO. 18 and a light chain variable region consisting of the amino acid sequence shown in SEQ ID NO. 19.

### (Labeled Antibody)

An antibody or an antigen binding fragment thereof comprising a heavy chain variable region consisting of the amino acid sequence shown in SEQ ID NO. 10 and a light chain variable region consisting of the amino acid sequence shown in SEQ ID NO. 11.

### (Solid Phase Antibody)

An antibody or an antigen binding fragment thereof comprising a heavy chain variable region consisting of the amino acid sequence shown in SEQ ID NO. 6 and a light chain variable region consisting of the amino acid sequence shown in SEQ ID NO. 7.

### (Labeled Antibody)

An antibody or an antigen binding fragment thereof comprising a heavy chain variable region consisting of the amino acid sequence shown in SEQ ID NO. 14 and a light chain variable region consisting of the amino acid sequence shown in SEQ ID NO. 15.

### (Solid Phase Antibody)

An antibody or an antigen binding fragment thereof comprising a heavy chain variable region consisting of the amino acid sequence shown in SEQ ID NO. 18 and a light chain variable region consisting of the amino acid sequence shown in SEQ ID NO. 19.

### (Labeled Antibody)

An antibody or an antigen binding fragment thereof comprising a heavy chain variable region consisting of the amino acid sequence shown in SEQ ID NO. 14 and a light chain variable region consisting of the amino acid sequence shown in SEQ ID NO. 15.

### (Solid Phase Antibody)

An antibody or an antigen binding fragment thereof comprising a heavy chain variable region consisting of the amino acid sequence shown in SEQ ID NO. 10 and a light chain variable region consisting of the amino acid sequence shown in SEQ ID NO. 11.

### (Labeled Antibody)

An antibody or an antigen binding fragment thereof comprising a heavy chain variable region consisting of the amino acid sequence shown in SEQ ID NO. 18 and a light chain variable region consisting of the amino acid sequence shown in SEQ ID NO. 19.

### (Solid Phase Antibody)

An antibody or an antigen binding fragment thereof comprising a heavy chain variable region consisting of the amino acid sequence shown in SEQ ID NO. 14 and a light chain variable region consisting of the amino acid sequence shown in SEQ ID NO. 15.

### (Labeled Antibody)

An antibody or an antigen binding fragment thereof comprising a heavy chain variable region consisting of the amino acid sequence shown in SEQ ID NO. 18 and a light chain variable region consisting of the amino acid sequence shown in SEQ ID NO. 19.

Those skilled in the art should recognize that as long as it is not technically contradicting, any one or more of any and all aspects described herein may be appropriately combined to carry out the present disclosure. Further, those skilled in the art should recognize that as long as it is not technically contradicting, and it is preferred that any and all preferred or advantageous aspects described herein are appropriately combined to carry out the present disclosure.

All of the disclosures of the literatures cited herein should be deemed as clearly cited herein by reference, and those skilled in the art can cite and recognize the related disclosed contents in these literatures as a part of the present specification according to the context herein without departing from the spirit and scope of the present disclosure.

The literatures cited herein are provided solely for the purpose of disclosing related technology preceding the filing date of the present application, and are not to be construed as an admission by the present inventors that the present invention does not hold the right to precede the disclosures due to prior inventions or for any other reason. All descriptions in these literatures are based on the information available to the present applicants, and do not configure in any way an admission that the contents of these descriptions are accurate.

The terms used herein are employed for describing particular embodiments, and do not intend to limit the invention.

The term "comprise" employed herein, unless the content clearly indicates to be understood otherwise, intends the presence of the described items (such as components, steps, elements, or numbers), and does not exclude the presence of other items (such as components, steps, elements, and numbers). The term "consist of" encompasses the aspects described with the terms "consist of" and/or "consist essentially of".

Unless otherwise defined, all terms used herein (including technical and scientific terms) have the same meaning as that broadly recognized by those skilled in the art of the technology to which the present disclosure belongs. The terms used herein, unless explicitly defined otherwise, should be construed as having meanings consistent with the meanings herein and in related technical fields, shall not be construed as having idealized or excessively formal meanings.

Terms such as first and second are employed to express various elements, and it should be recognized that these elements are not to be limited by these terms per se. These terms are employed solely for the purpose of discriminating one element from another, and for example, it is possible to describe a first element as a second element, and similarly, to describe a second element as a first element without departing from the scope of the present disclosure.

In the present specification, The numeric values employed for indicating a component content or a numeric value range and the like, unless explicitly indicated, are to be understood as being modified by the term "about". For example, "4°C", unless explicitly indicated, is recognized to mean "about 4°C", and it is natural that those skilled in the art can reasonably recognize the extent thereof according to technical common sense and the meaning of the present specification.

Unless clearly indicated to mean otherwise in context, when used in the specification and claims herein, it should be recognized that each aspect represented in singular form may also be a plural form as long as it is not technically contradicting, and vice versa.

The present disclosure will now be described in further detail with reference to Examples. However, the present disclosure can be embodied by various aspects, and shall not be construed as being limited to the Examples described herein. Those skilled in the art of related technical fields can carry out the present disclosure without changing the spirit and scope of the present disclosure with various modifications, additions, deletions, substitution, and the like.

### Examples

### Example 1: Production of Anti-Human EphA4 Rabbit Monoclonal Antibody

In order to produce a monoclonal antibody that binds to human EphA4 (Genbank Accession No. NP_004429.1, SEQ ID NO. 1), a protein having a secretory alkaline phosphatase (SEAP) and a histidine tag fused to the extracellular region of human EphA4 (positions 20 - 547) (SEQ ID NO. 2) (hereinafter referred to as "human EphA4 extracellular domain-SEAP-His protein", SEQ ID NO.3), a protein having a histidine tag fused to the extracellular domain of human EphA (positions 20 - 547) (SEQ ID NO. 2) (hereinafter referred to as "human EpA4extracellular domain-His protein", SEQ ID NO. 4), as well as a protein having a maltose-binding protein (MBP) and a histidine tag fused to the extracellular domain of human EphA4 (hereinafter referred to as "human EphA4 extracellular domain-MBP-His protein", SEQ ID NO. 5) were prepared by the following steps.

To begin with, a pcDNA 3.4-human EphA4 extracellular domain-SEAP-His expression vector, a pcDNA 3.4-human EphA4 extracellular domain-His expression vector, and a pcDNA 3.4-human EphA4 extracellular domain-MBP-His expression vector were constructed. Synthesis of a gene encoding SEAP-His and the human EphA4 extracellular domain was performed with Genscript. First, the synthesized gene fragment encoding SEAP-His was cloned into a pcDNA 3.4 vector (Invitrogen/LifeTechnologies) . The synthesized gene fragment of human EphA4 extracellular domain was cloned into the constructed pcDNA 3.4-SEAP-His expression vector to construct human EphA4 extracellular domain-SEAP-His expression vector. The pcDNA 3.4-human EphA4 extracellular domain-His expression vector was constructed by cloning the synthesized gene fragment of human EphA4 extracellular domain into a pcDNA 3.4 vector (Invitrogen/LifeTechnologies) having the DNA sequence encoding a histidine tag. The pcDNA 3.4-human EphA4 extracellular domain-MBP-His expression vector was constructed by amplifying the DNA sequence encoding the signal sequence and extracellular domain of human EphA4 by PCR, and then cloning into a pcDNA 3.4 vector (Invitrogen/LifeTechnologies) having the DNA sequence encoding a MBP and a histidine tag. Each of the above expression vectors was transfected to Expi293F cells (Thermo SCIENTIFIC) with the Expi293 expression system (Thermo SCIENTIFIC). The culture medium was collected, and the cells were removed and clarified. TALON resin (TaKaRa) was employed to perform purification, and the buffer was exchanged to PBS (FUJIFILM Wako) by dialysis or desalting column (Thermo SCIENTIFIC).

Following conventional means, the prepared human EphA4 extracellular domain-SEAP-His protein was used with an adjuvant to immunize a rabbit (IBL). After immunization, total RNA was prepared from the collected lymph node cells with RNeasy (QIAGEN), and treated with DNase (QIAGEN, RNase free DNase set). Reverse transcription products were prepared from said total RNA with RNA PCR kit (TAKARA) . Using the reverse transcription products obtained as the template, rabbit antibody phage library was constructed. Human EphA4 protein, human EphA4 antibody, and rabbit antibody phage library were employed to carry out screening, and rabbit antibody fragments (scFv) that specifically bind to human EphA4 were obtained. The human EphA4 extracellular domain-MBP-His protein and the human EphA4 antibody were captured onto Dynabeads (Thermo SCIENTIFIC), rabbit antibody phage library was added thereto, and after one or two hours, unbound phage was removed using PBS-Tween (0.1% v/v) or PBS by a series of wash cycles. After eluting the bound phage particles, this was amplified via infection of E. coli TG1 host cells. The infected TG1 cells were collected, plated on a plate, and this was incubated at 30°C. This panning was further performed with the amplified phage.

After second panning, a single colony from TG1 cells infected with the concentrated phage was employed to inoculate the medium in a 96-well plate. IPTG was added to induce expression of scFv + FLAG tag, and this was cultured with shaking at 30°C overnight. The TG1 cells were spin-downed, and wells having reactivity against human EphA4 were picked up with E. coli culture supernatant comprising scFv.

Reactivity against human EphA4 was evaluated by ELISA employing human EphA4 extracellular domain-MBP-His protein according to the following steps. Anti-FLAG antibodies (SIGMA) were coated on the wells of a 96-well plate (Thermo SCIENTIFIC). After incubating at 4°C overnight, the wells were blocked at room temperature for 2 hours with 2% skim milk (BD) . After washing three times with 0.02% Tween 20/PBS, human EphA4 extracellular domain-MBP-His protein (final concentration 20 nM) and E. coli culture supernatant comprising scFv were added to each well, and this was incubated at room temperature for 2 hours. After washing three times, horseradish peroxidase labeled anti-His antibody (MBL) was added, and this was incubated at room temperature for 1 hour. After washing five times, TMBZ (3,3',5,5'-tetramethylbenzidine, KPL) solution was added to the wells, and this was incubated for 15 - 20 minutes at room temperature. Equal amounts of quenching solution (2 N H₂SO₄, FUJIFILM Wako) were added to the wells, and absorbance at 450 nm was read with a microplate reader (Thermo SCIENTIFIC) . As a result of screening, human EphA4-specific rabbit antibody fragments were selected, and sequencing was performed to determine the gene sequences of each fragment.

DNA sequences encoding the variable regions of the rabbit antibody fragments (scFvs) obtained were respectively subcloned into vectors expressing the antibody heavy chain and light chain constant region to reformat the clones from scFv into IgG form. Expression vectors (pcDNA 3.4) comprising the gene sequence encoding the anti-human EphA4 rabbit monoclonal antibodies were produced. The amino acid sequence of the heavy chain variable region of KPEP11_04 is the amino acid sequence shown in SEQ ID NO. 6, and the amino acid sequence of the light chain variable region is the amino acid sequence shown in SEQ ID NO. 7. As the gene sequence encoding the amino acid sequence of KPEP11 04, the nucleic acid sequence shown in SEQ ID NO. 8 was employed for heavy chain variable region, and the nucleic acid sequence shown in SEQ ID NO. 9 was employed for light chain variable region. The amino acid sequence of the heavy chain variable region of KPEP11_08 is the amino acid sequence shown in SEQ ID NO. 10, and the amino acid sequence of the light chain variable region is the amino acid sequence shown in SEQ ID NO. 11. As the gene sequence encoding the amino acid sequence of KPEP11_08, the nucleic acid sequence shown in SEQ ID NO. 12 was employed for heavy chain variable region, and the nucleic acid sequence shown in SEQ ID NO. 13 was employed for light chain variable region. The amino acid sequence of the heavy chain variable region of KPEP11_10 is the amino acid sequence shown in SEQ ID NO. 14, and the amino acid sequence of the light chain variable region is the amino acid sequence shown in SEQ ID NO. 15. As the gene sequence encoding the amino acid sequence of KPEP11_10, the nucleic acid sequence shown in SEQ ID NO. 16 was employed for heavy chain variable region, and the nucleic acid sequence shown in SEQ ID NO. 17 was employed for light chain variable region. The amino acid sequence of the heavy chain variable region of KPEP11_18 is the amino acid sequence shown in SEQ ID NO. 18, and the amino acid sequence of the light chain variable region is the amino acid sequence shown in SEQ ID NO. 19. As the gene sequence encoding the amino acid sequence of KPEP11_18, the nucleic acid sequence shown in SEQ ID NO. 20 was employed for heavy chain variable region, and the nucleic acid sequence shown in SEQ ID NO. 21 was employed for light chain variable region. As the heavy chain constant regions of KPEP11_04, KPEP11_08, KPEP11_10, and KPEP11_18, the constant region of rabbit IgG (SEQ ID NO. 22) was employed. As the light chain constant region of KPEP11_04, KPEP11_08, KPEP11_10, and KPEP11_18, rabbit Igκ (SEQ ID NO. 23) was employed. The amino acid sequence of the full-length heavy chain (without the signal sequence) of KPEP11_04 is the amino acid sequence shown in SEQ ID NO. 24, and the amino acid sequence of the full-length light chain (without the signal sequence) is the amino acid sequence shown in SEQ ID NO. 25. The nucleic acid sequence encoding the full-length heavy chain of KPEP11_04 is the nucleic acid sequence shown in SEQ ID NO. 26, and the nucleic acid sequence encoding the full-length light chain is the nucleic acid sequence shown in SEQ ID NO. 27. The amino acid sequence of the full-length heavy chain (without the signal sequence) of KPEP11_08 is the amino acid sequence shown in SEQ ID NO. 28, and the amino acid sequence of the full-length light chain (without the signal sequence) is the amino acid sequence shown in SEQ ID NO. 29. The nucleic acid sequence encoding the full-length heavy chain of KPEP11_08 is the nucleic acid sequence shown in SEQ ID NO. 30, and the nucleic acid sequence encoding the full-length light chain is the nucleic acid sequence shown in SEQ ID NO. 31. The amino acid sequence of the full-length heavy chain (without the signal sequence) of KPEP11_10 is the amino acid sequence shown in SEQ ID NO. 32, and the amino acid sequence of the full-length light chain (without the signal sequence) is the amino acid sequence shown in SEQ ID NO. 33. The nucleic acid sequence encoding the full-length heavy chain of KPEP11_10 is the nucleic acid sequence shown in SEQ ID NO. 34, and the nucleic acid sequence encoding the full-length light chain is the nucleic acid sequence shown in SEQ ID NO. 35. The amino acid sequence of the full-length heavy chain (without the signal sequence) of KPEP11_18 is the amino acid sequence shown in SEQ ID NO. 36, and the amino acid sequence of the full-length light chain (without the signal sequence) is the amino acid sequence shown in SEQ ID NO. 37. The nucleic acid sequence encoding the full-length heavy chain of KPEP11_18 is the nucleic acid sequence shown in SEQ ID NO. 38, and the nucleic acid sequence encoding the full-length light chain is the nucleic acid sequence shown in SEQ ID NO. 39. These vectors were transfected to Expi293F cells (ThermoFisher) . The supernatant was collected, and anti-human EphA4 rabbit monoclonal antibodies were obtained by using MabSelect^{™} (Cytiva), MabSelect SuRepcc (Cytiva), or AmsphereA3 (JSR).

The CDRs of KPEP11_04, KPEP11_08, KPEP11_10, and KPEP11_18 were determined according to the Kabat definition for identifying CDR. The amino acid sequence and the nucleic acid sequence of the CDR of KPEP11_04 are respectively shown in Table 1 and Table 2. The amino acid sequence and the nucleic acid sequence of the CDR of KPEP11_08 and KPEP11_10 are respectively shown in Table 3 and Table 4. The amino acid sequence and the nucleic acid sequence of the CDR of KPEP11_18 are respectively shown in Table 5 and Table 6.

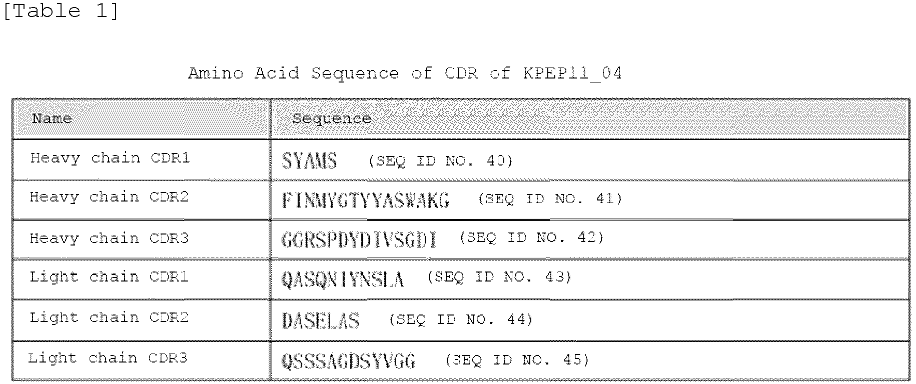

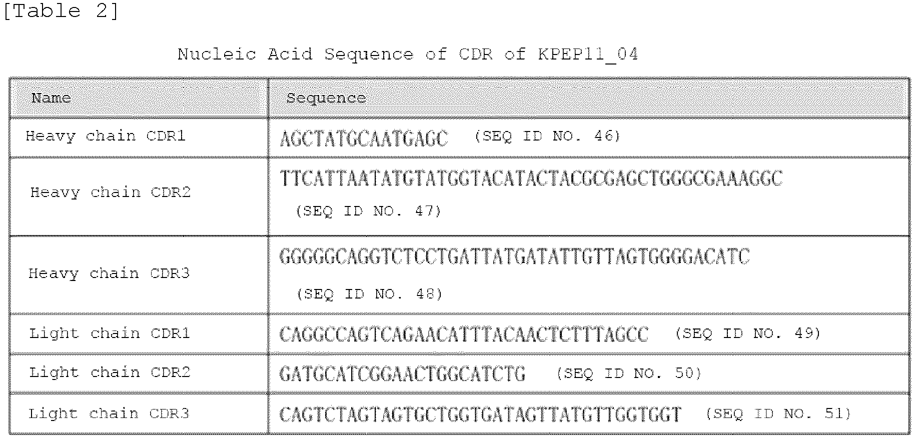

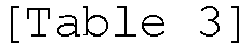

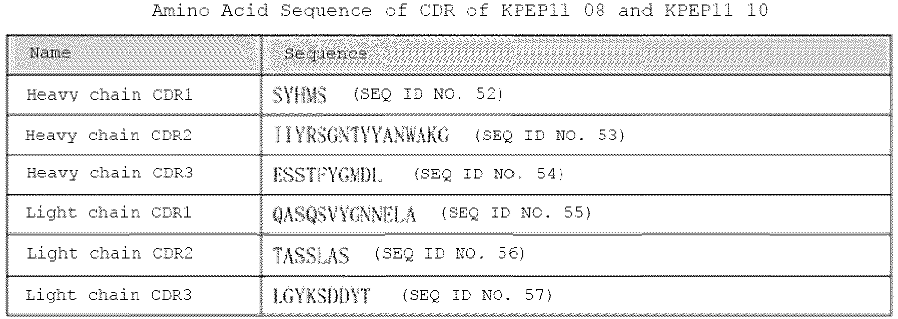

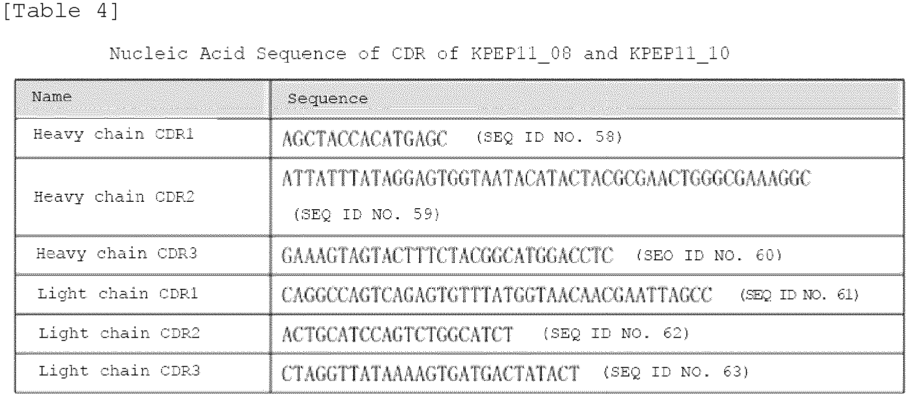

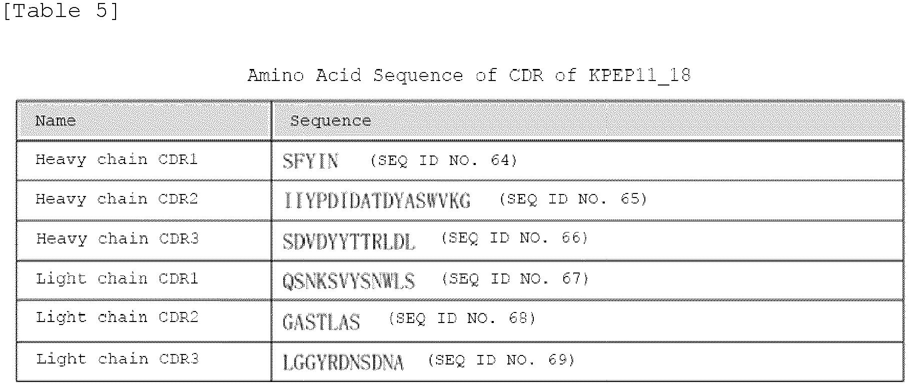

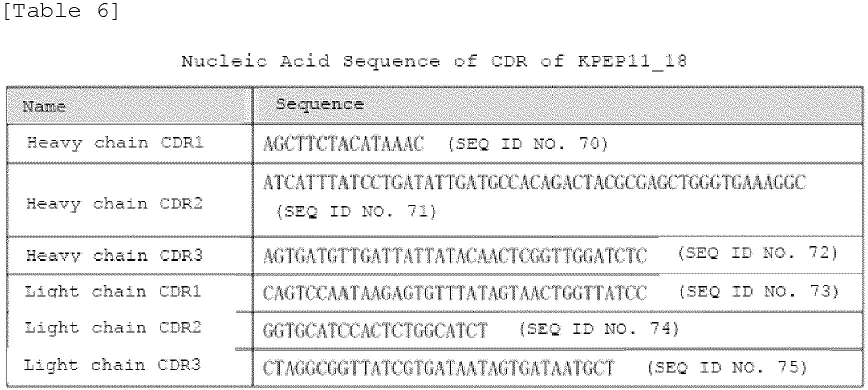

With the same method as the above production method, anti-human EphA4 rabbit monoclonal antibodies KPEP11_01, KPEP11_02, KPEP11_05, KPEP11_07, KPEP11_09, KPEP11_12, KPEP11_13, and KPEP11_20 were produced. The amino acid sequence of the heavy chain variable region of KPEP11_01 is the amino acid sequence shown in SEQ ID NO. 76, and the amino acid sequence of the light chain variable region is the amino acid sequence shown in SEQ ID NO. 77. The amino acid sequence of the heavy chain variable region of KPEP11_02 is the amino acid sequence shown in SEQ ID NO. 78, and the amino acid sequence of the light chain variable region is the amino acid sequence shown in SEQ ID NO. 79. The amino acid sequence of the heavy chain variable region of KPEP11_05 is the amino acid sequence shown in SEQ ID NO. 80, and the amino acid sequence of the light chain variable region is the amino acid sequence shown in SEQ ID NO. 81. The amino acid sequence of the heavy chain variable region of KPEP11_07 is the amino acid sequence shown in SEQ ID NO. 82, and the amino acid sequence of the light chain variable region is the amino acid sequence shown in SEQ ID NO. 83. The amino acid sequence of the heavy chain variable region of KPEP11_09 is the amino acid sequence shown in SEQ ID NO. 84, and the amino acid sequence of the light chain variable region is the amino acid sequence shown in SEQ ID NO. 85. The amino acid sequence of the heavy chain variable region of KPEP11_12 is the amino acid sequence shown in SEQ ID NO. 86, and the amino acid sequence of the light chain variable region is the amino acid sequence shown in SEQ ID NO. 87. The amino acid sequence of the heavy chain variable region of KPEP11_13 is the amino acid sequence shown in SEQ ID NO. 88, and the amino acid sequence of the light chain variable region is the amino acid sequence shown in SEQ ID NO. 89. The amino acid sequence of the heavy chain variable region of KPEP11_20 is the amino acid sequence shown in SEQ ID NO. 90, and the amino acid sequence of the light chain variable region is the amino acid sequence shown in SEQ ID NO. 91. The constant region of rabbit IgG (SEQ ID NO. 22) was used as the heavy chain constant region of these antibodies. Further, rabbit Igκ (SEQ ID NO. 23) was used as the light chain constant region of these antibodies.

The CDR of each antibody produced was determined according to the Kabat definition for identifying CDR. The amino acid sequence of CDR of each antibody are respectively shown in Table 7 to Table 11.

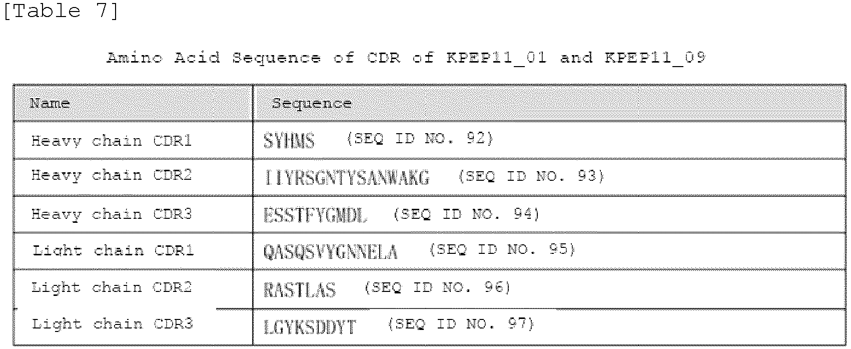

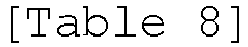

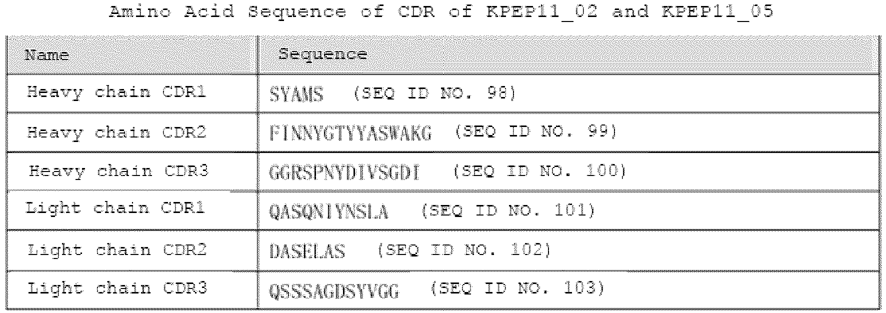

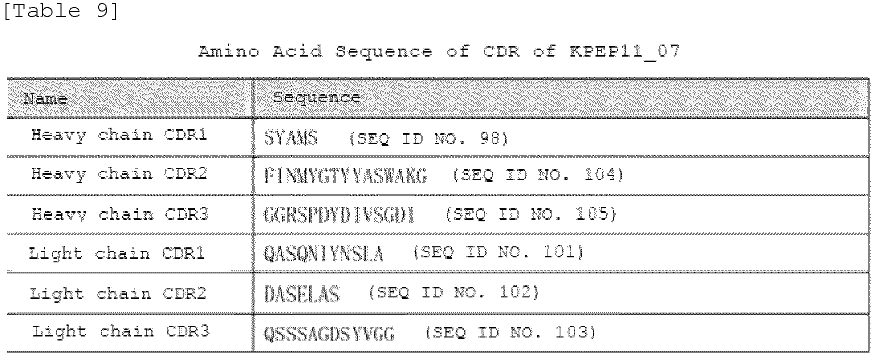

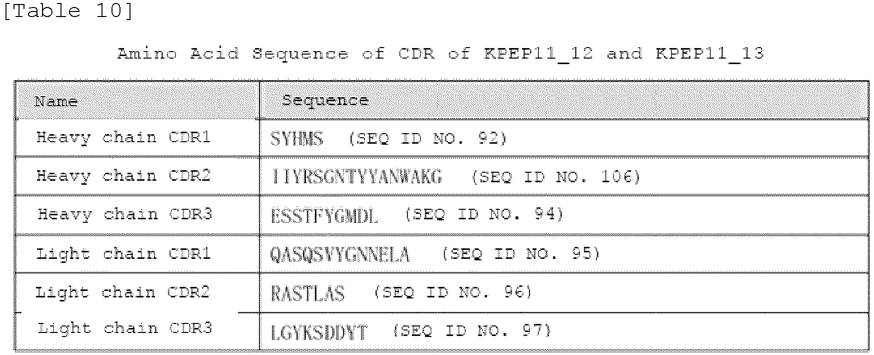

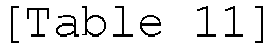

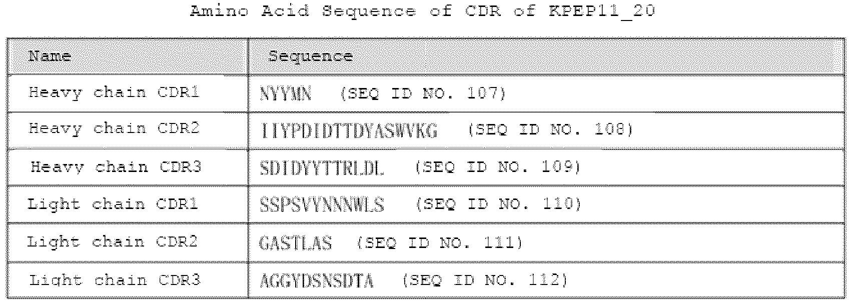

### Example 2: Selectivity of Anti-Human EphA4 Monoclonal Antibody Against Human Eph Receptor

For the anti-human EphA4 monoclonal antibodies produced in Example 1, evaluation of binding activity of human Eph receptor was performed according to the following steps. Mouse anti-6-His antibodies (R&D) were coated on the wells of a 96-well plate (Thermo SCIENTIFIC). After incubating at room temperature for 1 hour, the wells were blocked at room temperature for 1 hour with 1% Block Ace (KAC) . After washing three times with 0.02% Tween 20/PBS, each Eph receptor extracellular domain-His protein of human (Creative biomart, final concentration 1 nM) was seeded to each well, and this was incubated at room temperature for 1 hour. After washing three times, anti-human EphA4 rabbit monoclonal antibody (10 µg/mL) was added, and this was incubated at room temperature for 1 hour. After washing three times, horseradish peroxidase labeled goat anti-rabbit IgG polyclonal antibody (abcam) was added, and this was incubated at room temperature for 1 hour. After washing three times, TMBZ (3,3',5,5'-tetramethylbenzidine, KPL) solution was added to the wells, and this was incubated for four and a half minutes at room temperature. Equal amounts of quenching solution (2 N H₂SO₄, FUJIFILM Wako) were added to the wells, and absorbance at 450 nm and 650 nm were read with a microplate reader (Thermo SCIENTIFIC).

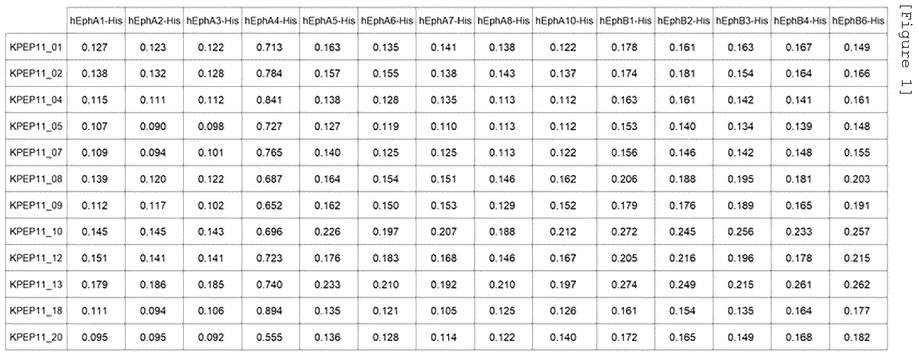

It was found that KPEP11_01, KPEP11_02, KPEP11_04, KPEP11_05, KPEP11_07, KPEP11_08, KPEP11_09, KPEP11_10, KPEP11_12, KPEP11_13, KPEP11_18, and KPEP11_20 specifically bind to human EphA4 among human Eph receptor family (Figure 1).

### Example 3: Reactivity of Anti-Human EphA4 Monoclonal Antibody Against Mouse, Rat, Rabbit, Monkey, and Human EphA4

For the anti-human EphA4 monoclonal antibodies produced in Example 1, evaluation of binding activity to various EphA4 was performed according to the following steps. Mouse anti-6-His antibodies (R&D) were coated on the wells of a 96-well plate (Thermo SCIENTIFIC). After incubating at room temperature for 1 hour, the wells were blocked at 4°C overnight with 1% Block Ace (KAC) . After washing three times with 0.02% Tween 20/PBS, the wells were seeded with mouse EphA4 extracellular domain-SEAP-His protein, rat EphA4 extracellular domain-SEAP-His protein, rabbit EphA4 extracellular domain-SEAP-His protein, monkey EphA4 extracellular domain-SEAP-His protein, human EphA4 extracellular domain-SEAP-His protein, or SEAP-His protein (final concentration 1 nM), and this was incubated at room temperature for 1 hour. After washing three times, anti-human EphA4 rabbit monoclonal antibody (10 µg/mL) was added, and this was incubated at room temperature for about 1 hour. After washing three times, horseradish peroxidase labeled goat anti-rabbit IgG polyclonal antibody (abcam) was added, and this was incubated at room temperature for 1 hour. After washing three times, TMBZ (3,3',5,5'-tetramethylbenzidine, KPL) solution was added to the wells, and this was incubated for 3 minutes at room temperature. Equal amounts of quenching solution (2 N H₂SO₄, FUJIFILM Wako) were added to the wells, and absorbance at 450 nm and 650 nm were read with a microplate reader (Thermo SCIENTIFIC).

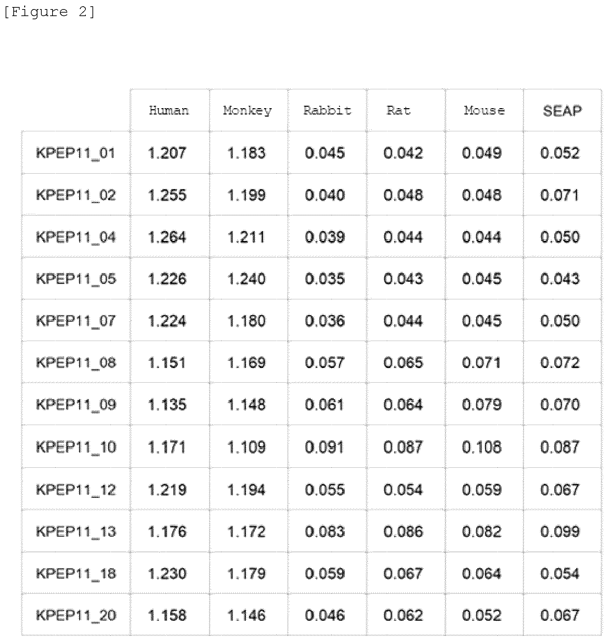

KPEP11_01, KPEP11_02, KPEP11_04, KPEP11_05, KPEP11_07, KPEP11_08, KPEP11_09, KPEP11_10, KPEP11_12, KPEP11_13, KPEP11_18, and KPEP11_20 had binding activity for monkey and human EphA4 (Figure 2) .

### Example 4: Reactivity of Anti-Human EphA4 Monoclonal Antibody Against Human EphA4 Extracellular Domain, Ligand Binding Domain, Fibronectin III-Type Domain 1, and Fibronectin III-Type Domain 2

For the anti-human EphA4 monoclonal antibodies produced in Example 1, evaluation of binding activity to various EphA4 was performed according to the following steps. Mouse anti-6-His antibodies (R&D) were coated on the wells of a 96-well plate (Thermo SCIENTIFIC). After incubating at room temperature for 1 hour, the wells were blocked at room temperature for 1 hour with 1% Block Ace (KAC) . After washing three times with 0.02% Tween 20/PBS, the wells were seeded with human EphA4 extracellular domain-MBP-His protein, human EphA4 ligand binding domain-MBP-His protein, human EphA4 fibronectin III-type domain 1-MBP-His protein, human EphA4 fibronectin III-type domain 2-MBP-His protein, or MBP-His protein (final concentration 1 nM), and this was incubated at room temperature for 1 hour. After washing three times, anti-human EphA4 rabbit monoclonal antibody (10 µg/mL) was added, and this was incubated at room temperature for 1 hour. After washing three times, horseradish peroxidase labeled goat anti-rabbit IgG polyclonal antibody (abcam) was added, and this was incubated at room temperature for 1 hour. After washing three times, TMBZ (3,3',5,5'-tetramethylbenzidine, KPL) solution was added to the wells, and this was incubated for four and a half minutes at room temperature. Equal amounts of quenching solution (2 N H₂SO₄, FUJIFILM Wako) were added to the wells, and absorbance at 450 nm and 650 nm were read with a microplate reader (Thermo SCIENTIFIC).

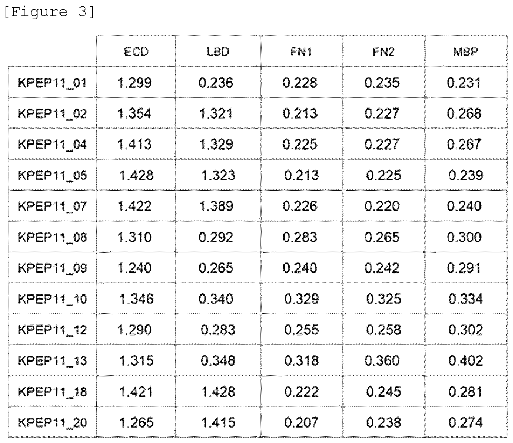

KPEP11_02, KPEP11_04, KPEP11_05, KPEP11_07, KPEP11_18, and KPEP11_20 had binding activity for human EphA4 extracellular domain (ECD) and ligand binding domain (LBD). KPEP11_01, KPEP11_08, KPEP11_09, KPEP11_10, KPEP11_12, and KPEP11_13 had binding activity form human EphA4 extracellular domain (ECD) (Figure 3).

### Example 5: Reactivity Against Human EphA4 Extracellular Domain by Sandwich ELISA

For the anti-human EphA4 monoclonal antibodies produced in Example 1, evaluation of binding activity against human EphA4 extracellular domain was performed according to the following steps. Each anti-human EphA4 monoclonal antibody was respectively prepared to a final concentration of 1 µg/mL with 50 mM Tris-HCl (pH 7.5)/0.1% sodium azide, and coated onto the wells of a 96-well plate (Nunc) at 100 µL each. After incubating at 4°C overnight, the wells were blocked at room temperature for 1 hour or more or at 4°C overnight with a blocking solution (50 mM Tris-HCl (pH 7.5) /150 mM NaCl/0.01% Tween 20/5% skim milk (FUJIFILM Wako)). Each anti-human EphA4 monoclonal antibody as detection antibody was respectively labeled with HRP (Horseradish Peroxidase) with Peroxidase Labeling Kit-NH2 (DOJINDO) according to the attached manual. After washing the blocked plate three times with the wash solution (50 mM Tris-HCl (pH 7.5)/150 mM NaCl/0.01% Tween 20), the wells were seeded with human EphA4 extracellular domain serially diluted with the sample diluent (50 mM Tris-HCl (pH 7.5) /150 mM NaCl/0.2% EDTA-3Na/4% PEG 6000/0.01% Tween 20/0.2% Proclin 150 (Sigma-Aldrich)/5% skim milk (FUJIFILM Wako)) (0, 1, and 10 ng/mL), and this was incubated at room temperature for 2 hours. After washing three times, HRP-labeled anti-human EphA4 monoclonal antibody diluted 1500-fold with the sample diluent was added at 100 µL each, and this was incubated at room temperature for 1 hour. After washing three times, TMBZ (3,3',5,5'-tetramethylbenzidine, KPL) solution was added to the wells, and this was incubated for 30 minutes at room temperature. Equal amounts of quenching solution (2 N H₂SO₄, FUJIFILM Wako) were added to the wells, and absorbance at 450 nm and 620 nm were read with a microplate reader (Molecular Device).

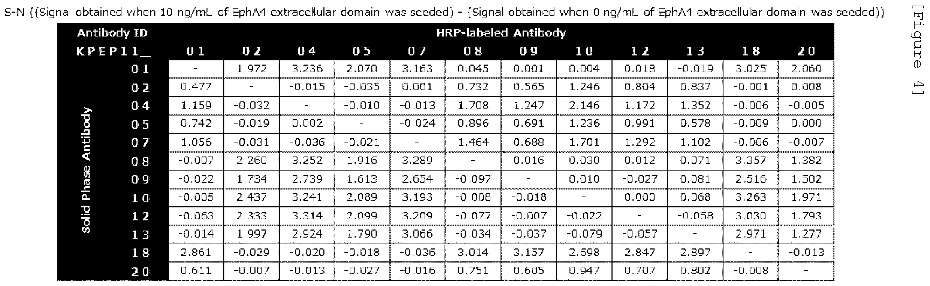

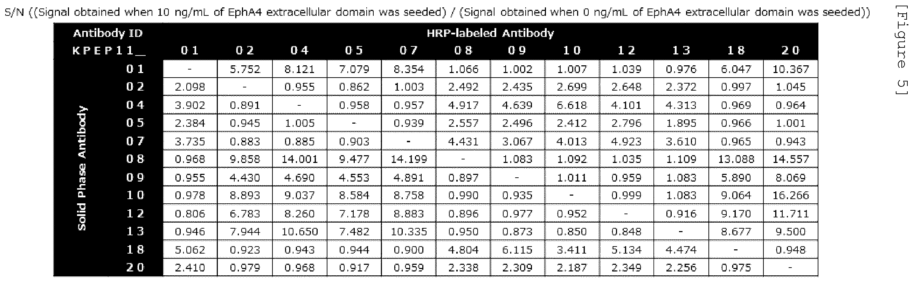

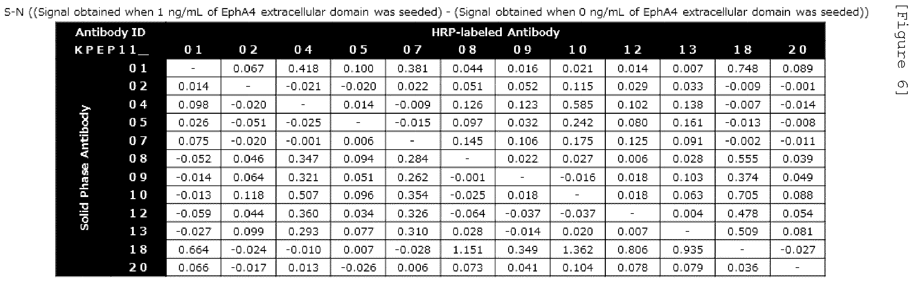

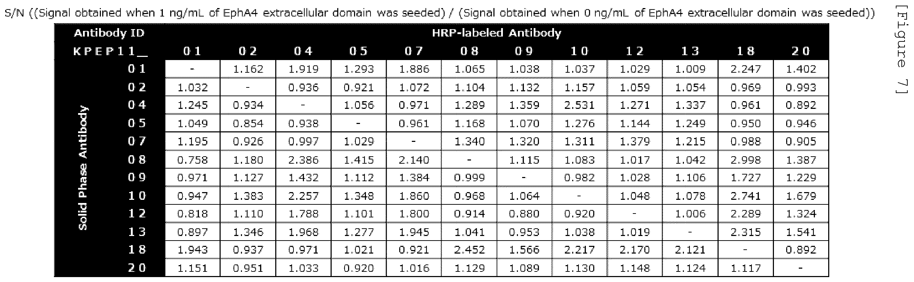

Lists of reactivity by a combination of each anti-human EphA4 monoclonal antibody are shown in Figure 4, Figure 5, Figure 6, and Figure 7, respectively as (1) S-N ((signal obtained when 10 ng/mL of EphA4 extracellular domain was seeded) - (signal obtained when 0 ng/mL of EphA4 extracellular domain was seeded)), (2) S/N ((signal obtained when 10 ng/mL of EphA4 extracellular domain was seeded) / (signal obtained when 0 ng/mL of EphA4 extracellular domain was seeded)), (3) S-N ((signal obtained when 1 ng/mL of EphA4 extracellular domain was seeded) - (signal obtained when 0 ng/mL of EphA4 extracellular domain was seeded)), and (4) S/N ((signal obtained when 1 ng/mL of EphA4 extracellular domain was seeded) / (signal obtained when 0 ng/mL of EphA4 extracellular domain was seeded)). Multiple combinations of anti-human EphA4 monoclonal antibodies showing strong binding activity against EphA4 extracellular domain were obtained.

### Example 6: Binding Affinity of Anti-Human EphA4 Monoclonal Antibody Against Human EphA4

The binding affinity of KPEP11_04, KPEP11_08, KPEP11_10, and KPEP11_18 against human EphA4 was determined by the surface plasmon resonance method (SPR method) employing Biacore T200 (Cytiva). First, an anti-His antibody (Cytiva, 28-9950-56) was diluted to 10 µg/mL with the immobilizing buffer (10 mM sodium acetate, pH 4.5), and immobilized onto a sensor chip CM5 according to the protocol attached to Biacore T200. Immobilization was performed by the amine coupling method employing N-hydroxysuccinimide (NHS) and N-ethyl-N'-(3-dimethylaminopropyl) carbodiimide hydrochloride salt (EDC), and ethanolamine was employed for blocking (sensor chips or immobilizing reagents were all from Cytiva). Human EphA4 extracellular domain-MBP-His 10 was diluted with a running buffer HBS-EP+ (Cytiva), and pumped onto a flow cell for 120 seconds to allow capturing (captured amount of about 3 RU). Subsequently, KPEP11 04, KPEP11_08, or KPEP11_10 serially diluted to the range of 50, 25, 12.5, 6.25, 3.125, 1.5625, and 0 nM, as well as KPEP11_18 serially diluted to the range of 25, 12.5, 6.25, 3.125, 1.5625, 0.78125, and 0 nM with HBS-EP+ were added to the chips for 120 seconds sensor chip, and the binding reaction curves at addition (binding phase, 120 seconds) and after completion of addition (dissociation phase, 300 seconds) were sequentially observed. At the end of each observation, 10 mM glycine hydrochloric acid pH 1.5 (60 seconds) and 3 M MgCl₂ (30 seconds) were added to regenerate the sensor chips. Fitting analysis by the 1:1 binding model employing the BIA evaluation software attached to the system was performed against the binding reaction curves obtained to calculate the binding affinity against human EphA4 (KD = kd/ka) . The experiment described above was carried out three times, and the mean was calculated for each parameter.

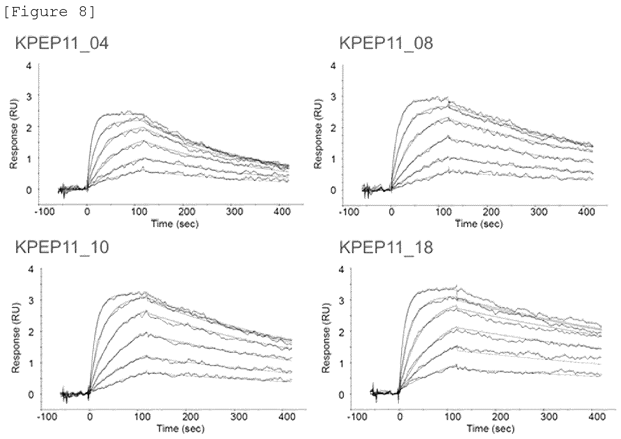

The binding affinity (KD value) of KPEP11_04, KPEP11_08, KPEP11_10, and KPEP11_18 against human EphA4 are respectively shown in Table 12, Table 13, Table 14, and Table 15. Moreover, a representative binding reaction curve is shown as Figure 8.

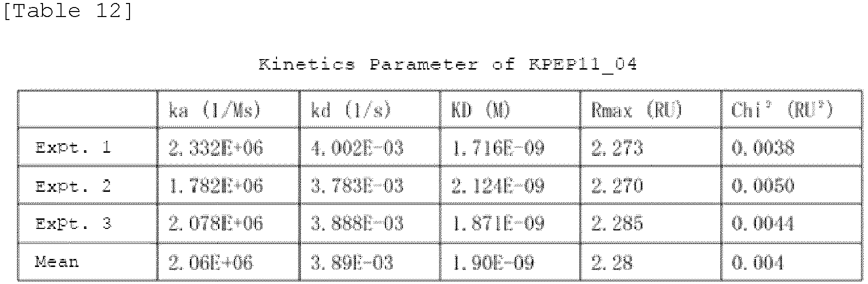

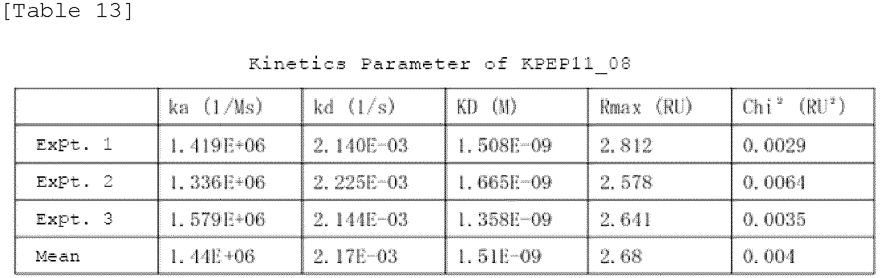

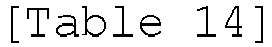

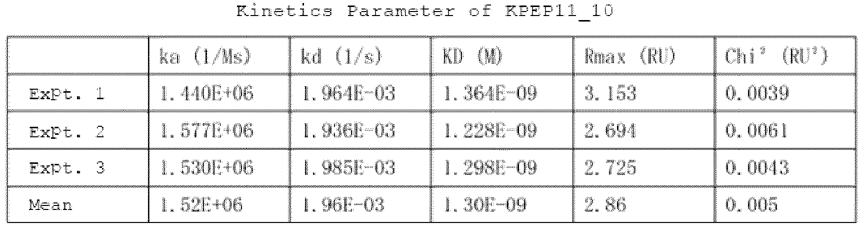

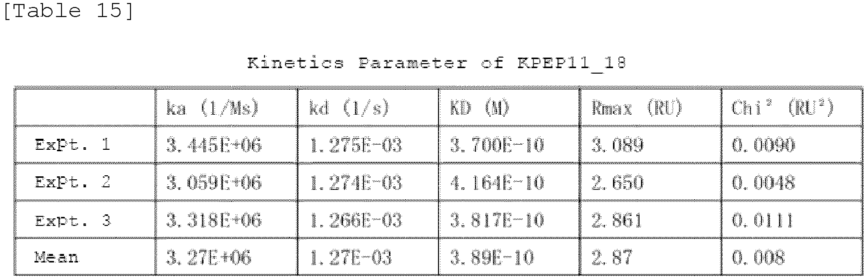

### Example 7: Selectivity of Anti-Human EphA4 Monoclonal Antibody Against Human Eph Receptor

For KPEP11_04, KPEP11_08, KPEP11_10, KPEP11_18, and anti-EphA4 polyclonal antibody (Sino Biological), evaluation of binding activity of human Eph receptor was performed according to the following steps. Mouse anti-6-His antibodies (R&D) were coated on the wells of a 96-well plate (Thermo SCIENTIFIC). After incubating at room temperature for 1 hour or at 4°C overnight, the wells were blocked at room temperature for 1 hour or at 4°C overnight with 1% Block Ace (KAC). After washing three times with 0.02% Tween 20/PBS, each Eph receptor extracellular domain-His protein of human (Creative biomart, final concentration 1 nM) was seeded to each well, and this was incubated at room temperature for 1 hour. After washing three times, KPEP11_04, KPEP11_08, KPEP11_10, KPEP11_18, or anti-EphA4 polyclonal antibody (1 µg/mL) was added, and this was incubated at room temperature for 1 hour. After washing three times, horseradish peroxidase labeled goat anti-rabbit IgG polyclonal antibody (abcam) was added, and this was incubated at room temperature for 1 hour. After washing five times, TMBZ

(3,3',5,5'-tetramethylbenzidine, KPL) solution was added to the wells, and this was incubated for 3-5 minutes at room temperature. Equal amounts of quenching solution (2 N H₂SO₄, FUJIFILM Wako) were added to the wells, and absorbance at 450 nm and 650 nm were read with a microplate reader (Thermo SCIENTIFIC).

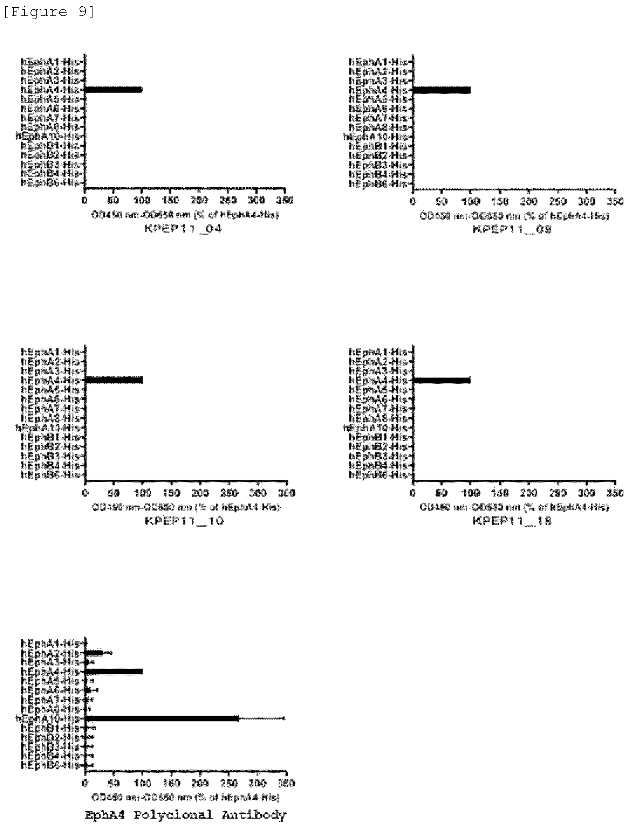

It was found that KPEP11_04, KPEP11_08, KPEP11_10, and KPEP11_18 specifically bind to human EphA4 among human Eph receptor family (Figure 9).

### Example 8: Reactivity of Anti-Human EphA4 Monoclonal Antibody Against Mouse, Rat, Rabbit, Monkey, and Human EphA4

The production of mouse EphA4 extracellular domain-SEAP-His protein, rat EphA4 extracellular domain-SEAP-His protein, rabbit EphA4 extracellular domain-SEAP-His protein, monkey EphA4 extracellular domain-SEAP-His protein, and human EphA4 extracellular domain-SEAP-His protein was performed according to the following steps. Synthesis of genes encoding SEAP-His as well as mouse EphA4 extracellular domain, rat EphA4 extracellular domain, rabbit EphA4 extracellular domain, monkey EphA4 extracellular domain, and human EphA4 extracellular domain was performed with Genscript. First, the synthesized gene encoding SEAP-His was cloned into a pcDNA 3.4 vector (Invitrogen/LifeTechnologies) . To the constructed pcDNA 3.4-SEAP-His expression vector, the synthesized genes of mouse EphA4 extracellular domain, rat EphA4 extracellular domain, rabbit EphA4 extracellular domain, monkey EphA4 extracellular domain, and human EphA4 extracellular domain were each cloned to construct mouse EphA4 extracellular domain-SEAP-His expression vector, rat EphA4 extracellular domain-SEAP-His expression vector, rabbit EphA4 extracellular domain-SEAP-His expression vector, monkey EphA4 extracellular domain-SEAP-His expression vector, and human EphA4 extracellular domain-SEAP-His expression vector. The amino acid sequence of human EphA4 utilized in vector construction is shown as SEQ ID NO. 1, the extracellular domain thereof as SEQ ID NO. 2, the amino acid sequence of monkey EphA4 as SEQ ID NO. 113, the extracellular domain thereof as SEQ ID NO. 114, the amino acid sequence of rabbit EphA4 as SEQ ID NO. 115, the extracellular domain thereof as SEQ ID NO. 116, the amino acid sequence of rat EphA4 as SEQ ID NO. 117, the extracellular domain thereof as SEQ ID NO. 118, the amino acid sequence of mouse EphA4 as SEQ ID NO. 119, and the extracellular domain thereof as SEQ ID NO. 120. Various EphA4 extracellular domain-SEAP-His proteins were prepared employing human EphA4 extracellular domain-SEAP-His protein expression vector, monkey EphA4 extracellular domain-SEAP-His protein expression vector, rabbit EphA4 extracellular domain-SEAP-His protein expression vector, rat EphA4 extracellular domain-SEAP-His protein expression vector, and mouse EphA4 extracellular domain-SEAP-His protein expression vector. The above expression vector was transfected to Expi293F cells (Thermo SCIENTIFIC) with the Expi293 expression system (Thermo SCIENTIFIC). After four days the culture medium was collected, and the cells were removed and clarified. TALON resin (TaKaRa) was used for purification, and the buffer was replaced with PBS (FUJIFILM Wako) by dialysis.

For KPEP11_04, KPEP11_08, KPEP11_10, KPEP11_18, and EphA4 polyclonal antibody (Sino Biological), evaluation of binding activity to various EphA4 was performed according to the following steps. Mouse anti-6-His antibodies (R&D) were coated on the wells of a 96-well plate (Thermo SCIENTIFIC). After incubating at room temperature for 1 hour or at 4°C overnight, the wells were blocked at room temperature for 1 hour or at 4°C overnight with 1% Block Ace (KAC) . After washing three times with 0.02% Tween 20/PBS, the wells were seeded with mouse EphA4 extracellular domain-SEAP-His protein, rat EphA4 extracellular domain-SEAP-His protein, rabbit EphA4 extracellular domain-SEAP-His protein, monkey EphA4 extracellular domain-SEAP-His protein, human EphA4 extracellular domain-SEAP-His protein, or SEAP-His protein (final concentration 1 nM), and this was incubated at room temperature for 1 hour. After washing three times, KPEP11_04, KPEP11_08, KPEP11_10, KPEP11_18, or EphA4 polyclonal antibody (0, 1.024e-6, 0.00000512, 0.0000256, 0.000128, 0.00064, 0.0032, 0.016, 0.08, 0.4, 2, and 10 µg/mL) was added, and this was incubated at room temperature for about 1 hour. After washing three times, horseradish peroxidase labeled goat anti-rabbit IgG polyclonal antibody (abcam) was added, and this was incubated at room temperature for 1 hour. After washing five times, TMBZ (3,3',5,5'-tetramethylbenzidine, KPL) solution was added to the wells, and this was incubated for 3 - 5 minutes at room temperature. Equal amounts of quenching solution (2 N H₂SO₄, FUJIFILM Wako) were added to the wells, and absorbance at 450 nm and 650 nm were read with a microplate reader (Thermo SCIENTIFIC).

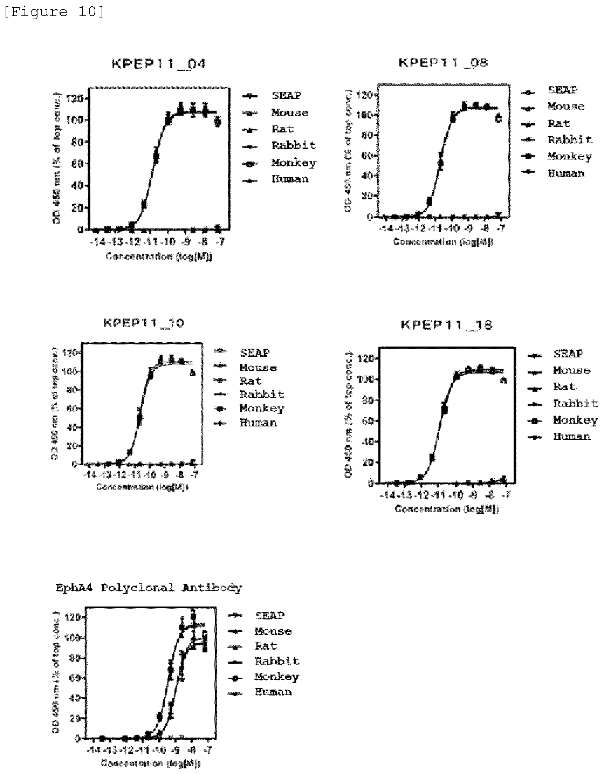

KPEP11_04, KPEP11_08, KPEP11_10, and KPEP11_18 had equivalent binding activity for monkey and human EphA4 (Figure 10).

### Example 9: Reactivity of Anti-Human EphA4 Monoclonal Antibody Against Human EphA4 Extracellular Domain, Ligand Binding Domain, Fibronectin III-Type Domain 1, and Fibronectin III-Type Domain 2

The production of proteins having a maltose-binding protein (MBP) and a histidine tag fused to the extracellular domain of human EphA4 (ECD), ligand binding domain (LBD), fibronectin III-type domain 1 (FN1), or fibronectin III-type domain 2 (FN2) (hereinafter referred to as "human EphA4 extracellular domain-MBP-His protein", "human EphA4 ligand binding domain-MBP-His protein", "human EphA4 fibronectin III-type domain 1-MBP-His protein", and "human EphA4 fibronectin III-type domain 2-MBP-His protein") was performed according to the following steps. To begin with, pcDNA 3.4-human EphA4 extracellular domain, ligand binding domain, fibronectin III-type domain 1, or fibronectin III-type domain 2-MBP-His expression vector were constructed. First, the DNA sequence encoding the signal sequence of human EphA4 (SEQ ID NO. 121) or the signal sequence of preprotrypsin (SEQ ID NO. 122) and each domain of human EphA4 was amplified by PCR, cloned into a pcDNA 3.4 vector (Invitrogen/LifeTechnologies) having a DNA sequence encoding a MBP and a histidine tag with a AAA or G4S linker, to construct expression vectors of human EphA4 extracellular domain-MBP-His protein, human EphA4 ligand binding domain-MBP-His protein, human EphA4 fibronectin III-type domain 1-MBP-His protein, and human EphA4 fibronectin III-type domain 2-MBP-His protein. The amino acid sequence of human EphA4 utilized in vector construction is shown as SEQ ID NO. 1, the extracellular domain thereof as SEQ ID NO. 2, the ligand binding domain as SEQ ID NO. 123, the fibronectin III-type domain 1 as SEQ ID NO. 124, the fibronectin III-type domain 2 as SEQ ID NO. 125, and the MBP and the histidine tag (MBP-His protein) as SEQ ID NO. 126. The above expression vector was transfected to Expi293F cells (Thermo SCIENTIFIC) with the Expi293 expression system (Thermo SCIENTIFIC). After four days the culture medium was collected, and the cells were removed and clarified. Human EphA4 extracellular domain-MBP-His protein or human EphA4 ligand binding domain-MBP-His protein were purified with TALON resin (TaKaRa), and the buffer was exchanged to PBS (FUJIFILM Wako) by dialysis. Human EphA4 fibronectin III-type domain 1-MBP-His protein and human EphA4 fibronectin III-type domain 2-MBP-His protein were purified with Amylose resin (NEB), and the monomer fraction was purified with AKTA Explore 10s/Superdex 200 10/300 GL (Cytiva).

For KPEP11_04, KPEP11_08, KPEP11_10, KPEP11_18, and EphA4 polyclonal antibody (Sino Biological), evaluation of binding activity to various EphA4 was performed according to the following steps. Mouse anti-6-His antibodies (R&D) were coated on the wells of a 96-well plate (Thermo SCIENTIFIC). After incubating at room temperature for 1 hour or at 4°C overnight, the wells were blocked at room temperature for 1 hour or at 4°C overnight with 1% Block Ace (KAC) . After washing three times with 0.02% Tween 20/PBS, the wells were seeded with human EphA4 extracellular domain-MBP-His protein, human EphA4 ligand binding domain-MBP-His protein, human EphA4 fibronectin III-type domain 1-MBP-His protein, human EphA4 fibronectin III-type domain 2-MBP-His protein, or MBP-His protein (final concentration 1 nM), and this was incubated at room temperature for 1 hour. After washing three times, KPEP11 04, KPEP11_08, KPEP11_10, KPEP11_18, or EphA4 polyclonal antibody (10 nM) was added, and this was incubated at room temperature for 1 hour. After washing three times, horseradish peroxidase labeled goat anti-rabbit IgG polyclonal antibody (abcam) was added, and this was incubated at room temperature for 1 hour. After washing three times, TMBZ (3,3',5,5'-tetramethylbenzidine, KPL) solution was added to the wells, and this was incubated for 3 - 5 minutes at room temperature. Equal amounts of quenching solution (2 N H₂SO₄, FUJIFILM Wako) were added to the wells, and absorbance at 450 nm and 650 nm were read with a microplate reader (Thermo SCIENTIFIC).

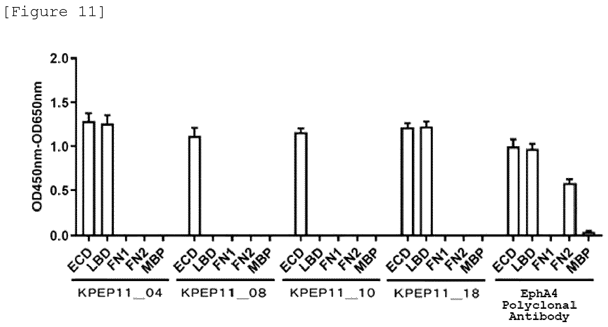

KPEP11_04 and KPEP11_18 had binding activity to human EphA4 extracellular domain (ECD) and ligand binding domain (LBD). KPEP11_08 and KPEP11_10 had binding activity to human EphA4 extracellular domain (ECD) (Figure 11).

### Example 10: Reactivity Against Human EphA4 Extracellular Domain and EphA4 N-Terminal Fragments in Human Cerebrospinal Fluid by Sandwich ELISA 1

For KPEP11_10, KPEP11_18, and EphA4 antibody (R&D), evaluation of binding activity against human EphA4 extracellular domain and EphA4 N-terminal fragments in human cerebrospinal fluid (CSF) was performed according to the following steps. KPEP11_10 was prepared to a final concentration of 1 µg/mL with 50 mM Tris-HCl (pH 7.5) /0.1% sodium azide, and coated onto the wells of a 96-well plate (Nunc) at 100 µL each. After incubating at 4°C overnight, the wells were blocked at room temperature for 1 hour or more or at 4°C overnight with a blocking solution (50 mM Tris-HCl (pH 7.5)/150 mM NaCl/0.01% Tween 20/5% skim milk (FUJIFILM Wako)). KPEP11_18 and EphA4 antibody (R&D) were labeled with HRP with Peroxidase Labeling Kit-NH2 (DOJINDO) according to the attached manual. After washing the blocked plate three times with the wash solution (50 mM Tris-HCl (pH 7.5)/150 mM NaCl/0.01% Tween 20), the wells were seeded with human EphA4 extracellular domain serially diluted with the sample diluent (50 mM Tris-HCl (pH 7.5) /150 mM NaCl/0.2% EDTA-3Na/4% PEG 6000/0.01% Tween 20/0.2% Proclin 150 (Sigma-Aldrich)/5% skim milk (FUJIFILM Wako)) (0, 0.156, 0.313, 0.625, 1.25, 2.5, 5, and 10 ng/mL, shown as EphA4 in the Figure), or a sample (human cerebrospinal fluid) diluted by 100-fold with the sample diluent, and incubated at room temperature for 2 hours. After washing five times, HRP-labeled KPEP11_18 or HRP-labeled EphA4 antibody (R&D) diluted by 10000-fold with the sample diluent were added at 100 µL each, and this was incubated at room temperature for 1 hour. After washing three times, TMBZ (3,3',5,5'-tetramethylbenzidine, KPL) solution was added to the wells, and this was incubated for 30 minutes at room temperature. Equal amounts of quenching solution (2 N H₂SO₄, FUJIFILM Wako) were added to the wells, and absorbance at 450 nm and 650 nm were read with a microplate reader (Molecular Device).

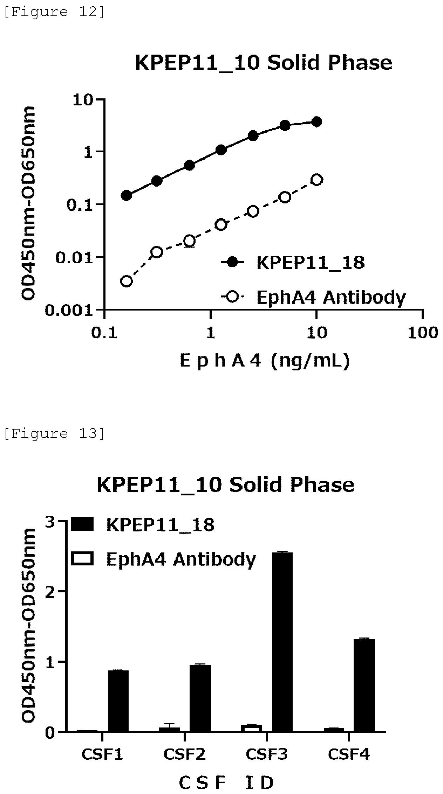

The result of evaluating reactivity against human EphA4 extracellular domain is shown in Figure 12. Sandwich ELISA constructed with KPEP11_10 and HRP-labeled KPEP11_18 had extremely high reactivity against human EphA4 extracellular domain compared to the measuring system constructed with KPEP11_10 and HRP-labeled EphA4 antibody (R&D). Next, the result of evaluating reactivity against EphA4 N-terminal fragments in the cerebrospinal fluid is shown in Figure 13. It was found that whereas almost no reaction signal was obtained with the measuring system constructed with KPEP11_10 and HRP-labeled EphA4 antibody (R&D), the measuring system constructed with KPEP11_10 and HRP-labeled KPEP11_18 showed extremely high reactivity.

### Example 11: Reactivity Against Human EphA4 Extracellular Domain and EphA4 N-Terminal Fragments in Human Cerebrospinal Fluid by Sandwich ELISA 2

For KPEP11_10, KPEP11_18, and EphA4 antibody (R&D), evaluation of binding activity against human EphA4 extracellular domain and EphA4 N-terminal fragments in human cerebrospinal fluid (CSF) was performed according to the following steps. KPEP11_18 was prepared to a final concentration of 1 µg/mL with 50 mM Tris-HCl (pH 7.5) /0.1% sodium azide, and coated onto the wells of a 96-well plate (Nunc) at 100 µL each. After incubating at 4°C overnight, the wells were blocked at room temperature for 1 hour or more or at 4°C overnight with a blocking solution (50 mM Tris-HCl (pH 7.5) /150 mM NaCl/0.01% Tween 20/5% skim milk (FUJIFILM Wako)). KPEP11_10 and EphA4 antibody (R&D) were labeled with HRP with Peroxidase Labeling Kit-NH2 (DOJINDO) according to the attached manual. After washing the blocked plate three times with the wash solution (50 mM Tris-HCl (pH 7.5)/150 mM NaCl/0.01% Tween 20), the wells were seeded with human EphA4 extracellular domain serially diluted with the sample diluent (50 mM Tris-HCl (pH 7.5) /150 mM NaCl/0.2% EDTA-3Na/4% PEG 6000/0.01% Tween 20/0.2% Proclin 150 (Sigma-Aldrich)/5% skim milk (FUJIFILM Wako)) (0, 0.156, 0.313, 0.625, 1.25, 2.5, 5, and 10 ng/mL, shown as EphA4 in the Figure), or a sample (human cerebrospinal fluid) diluted by 100-fold, and incubated at room temperature for 2 hours. After washing five times, HRP-labeled KPEP11 10 or HRP-labeled EphA4 antibody (R&D) diluted by 10000-fold with the sample diluent were added at 100 µL each, and this was incubated at room temperature for 1 hour. After washing three times, TMBZ (3,3',5,5'-tetramethylbenzidine, KPL) solution was added to the wells, and this was incubated for 30 minutes at room temperature. Equal amounts of quenching solution (2 N H₂SO₄, FUJIFILM Wako) were added to the wells, and absorbance at 450 nm and 650 nm were read with a microplate reader (Molecular Device).

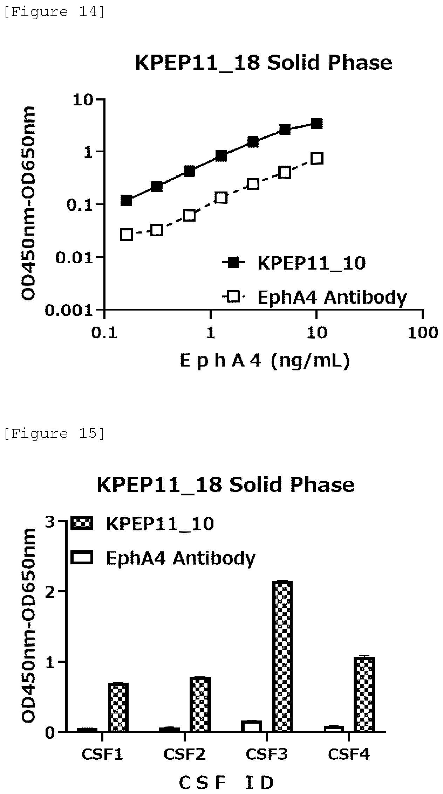

The result of evaluating reactivity against human EphA4 extracellular domain is shown in Figure 14. Sandwich ELISA constructed with KPEP11_18 and HRP-labeled KPEP11_10 had extremely high reactivity against human EphA4 extracellular domain compared to the measuring system constructed with KPEP11_18 and HRP-labeled EphA4 antibody (R&D). Next, the result of evaluating reactivity against EphA4 N-terminal fragments in the cerebrospinal fluid is shown in Figure 15. It was found that whereas almost no reaction signal was obtained with the measuring system constructed with KPEP11_18 and HRP-labeled EphA4 antibody (R&D), the measuring system constructed with KPEP11_18 and HRP-labeled KPEP11_10 showed extremely high reactivity.

### Example 12: Reactivity Against Human EphA4 Extracellular Domain and EphA4 N-Terminal Fragments in Human Cerebrospinal Fluid by Sandwich ELISA 3

For KPEP11_10, KPEP11_04, and EphA4 antibody (R&D), evaluation of binding activity against human EphA4 extracellular domain and EphA4 N-terminal fragments in human cerebrospinal fluid (CSF) was performed according to the following steps. KPEP11_10 was prepared to a final concentration of 1 µg/mL with 50 mM Tris-HCl (pH 7.5) /0.1% sodium azide, and coated onto the wells of a 96-well plate (Nunc) at 100 µL each. After incubating at 4°C overnight, the wells were blocked at room temperature for 1 hour or more or at 4°C overnight with a blocking solution (50 mM Tris-HCl (pH 7.5) /150 mM NaCl/0.01% Tween 20/5% skim milk (FUJIFILM Wako)). KPEP11_04 and EphA4 antibody (R&D) were labeled with HRP with Peroxidase Labeling Kit-NH2 (DOJINDO) according to the attached manual. After washing the blocked plate three times with the wash solution (50 mM Tris-HCl (pH 7.5)/150 mM NaCl/0.01% Tween 20), the wells were seeded with human EphA4 extracellular domain serially diluted with the sample diluent (50 mM Tris-HCl (pH 7.5) /150 mM NaCl/0.2% EDTA-3Na/4% PEG 6000/0.01% Tween 20/0.2% Proclin 150 (Sigma-Aldrich)/5% skim milk (FUJIFILM Wako)) (0, 0.156, 0.313, 0.625, 1.25, 2.5, 5, and 10 ng/mL, shown as EphA4 in the Figure), or a sample (human cerebrospinal fluid) diluted by 100-fold, and incubated at room temperature for 2 hours. After washing five times, HRP-labeled KPEP11_04 or HRP-labeled EphA4 antibody (R&D) diluted by 10000-fold with the sample diluent were added at 100 µL each, and this was incubated at room temperature for 1 hour. After washing three times, TMBZ (3,3',5,5'-tetramethylbenzidine, KPL) solution was added to the wells, and this was incubated for 30 minutes at room temperature. Equal amounts of quenching solution (2 N H₂SO₄, FUJIFILM Wako) were added to the wells, and absorbance at 450 nm and 650 nm were read with a microplate reader (Molecular Device).

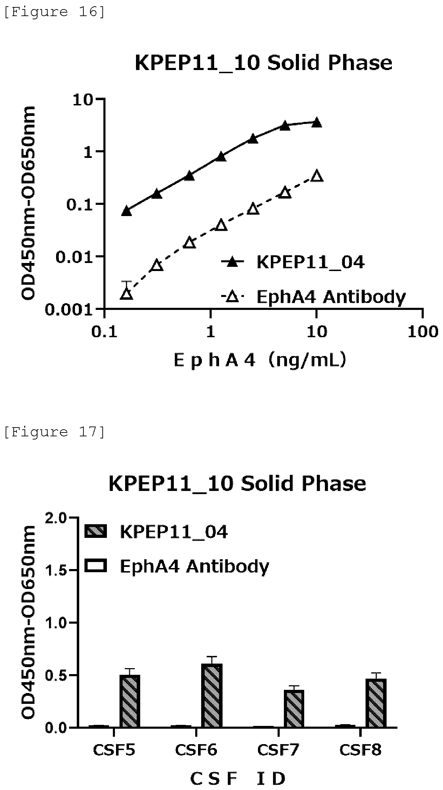

The result of evaluating reactivity against human EphA4 extracellular domain is shown in Figure 16. Sandwich ELISA constructed with KPEP11_10 and HRP-labeled KPEP11_04 had extremely high reactivity against human EphA4 extracellular domain compared to the measuring system constructed with KPEP11_10 and HRP-labeled EphA4 antibody (R&D). Next, the result of evaluating reactivity against EphA4 N-terminal fragments in the cerebrospinal fluid is shown in Figure 17. It was found that whereas almost no reaction signal was obtained with the measuring system constructed with KPEP11_10 and HRP-labeled EphA4 antibody (R&D), the measuring system constructed with KPEP11_10 and HRP-labeled KPEP11_04 showed extremely high reactivity.

### Example 13: Reactivity Against Human EphA4 Extracellular Domain and EphA4 N-Terminal Fragments in Human Cerebrospinal Fluid by Sandwich ELISA 4

For KPEP11_08, KPEP11_18, and EphA4 antibody (R&D), evaluation of binding activity against human EphA4 extracellular domain and EphA4 N-terminal fragments in human cerebrospinal fluid (CSF) was performed according to the following steps. KPEP11_18 was prepared to a final concentration of 1 µg/mL with 50 mM Tris-HCl (pH 7.5) /0.1% sodium azide, and coated onto the wells of a 96-well plate (Nunc) at 100 µL each. After incubating at 4°C overnight, the wells were blocked at room temperature for 1 hour or more or at 4°C overnight with a blocking solution (50 mM Tris-HCl (pH 7.5) /150 mM NaCl/0.01% Tween 20/5% skim milk (FUJIFILM Wako)). KPEP11_08 and EphA4 antibody (R&D) were labeled with HRP with Peroxidase Labeling Kit-NH2 (DOJINDO) according to the attached manual. After washing the blocked plate three times with the wash solution (50 mM Tris-HCl (pH 7.5)/150 mM NaCl/0.01% Tween 20), the wells were seeded with human EphA4 extracellular domain serially diluted with the sample diluent (50 mM Tris-HCl (pH 7.5) /150 mM NaCl/0.2% EDTA-3Na/4% PEG 6000/0.01% Tween 20/0.2% Proclin 150 (Sigma-Aldrich)/5% skim milk (FUJIFILM Wako)) (0, 0.156, 0.313, 0.625, 1.25, 2.5, 5, and 10 ng/mL, shown as EphA4 in the Figure), or a sample (human cerebrospinal fluid) diluted by 100-fold, and incubated at room temperature for 2 hours. After washing five times, HRP-labeled KPEP11_08 or HRP-labeled EphA4 antibody (R&D) diluted by 10000-fold with the sample diluent was added at 100 µL each, and this was incubated at room temperature for 1 hour. After washing three times, TMBZ (3,3',5,5'-tetramethylbenzidine, KPL) solution was added to the wells, and this was incubated for 30 minutes at room temperature. Equal amounts of quenching solution (2 N H₂SO₄, FUJIFILM Wako) were added to the wells, and absorbance at 450 nm and 650 nm were read with a microplate reader (Molecular Device).

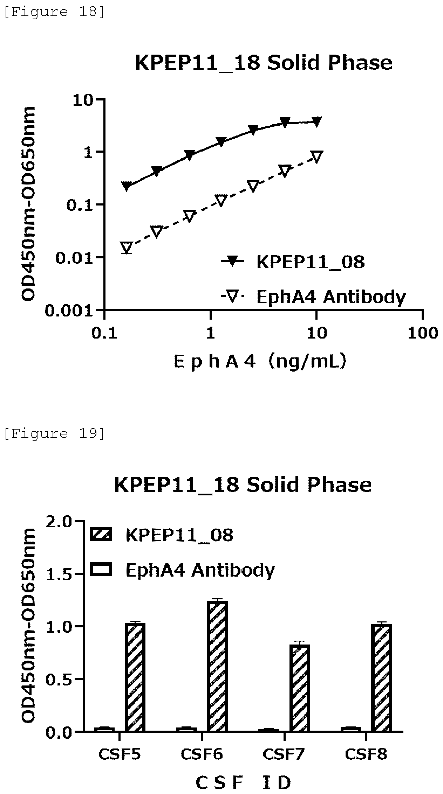

The result of evaluating reactivity against human EphA4 extracellular domain is shown in Figure 18. Sandwich ELISA constructed with KPEP11_18 and HRP-labeled KPEP11_08 had extremely high reactivity against human EphA4 extracellular domain compared to the measuring system constructed with KPEP11_18 and HRP-labeled EphA4 antibody (R&D). Next, the result of evaluating reactivity against EphA4 N-terminal fragments in the cerebrospinal fluid is shown in Figure 19. It was found that whereas almost no reaction signal was obtained with the measuring system constructed with KPEP11_18 and HRP-labeled EphA4 antibody (R&D), the measuring system constructed with KPEP11_18 and HRP-labeled KPEP11_08 showed extremely high reactivity.

### Example 14: Reactivity Against Human EphA4 Extracellular Domain, EphA4 N-Terminal Fragments in Human Plasma, and EphA4 N-Terminal Fragments in Human Cerebrospinal Fluid by Sandwich ELISA 5

For KPEP11_04, KPEP11_10, KPEP11_18, EphA4 antibody (Sino Biological, hereinafter shown as "EphA4 antibody (Sino)"), and EphA4 antibody (R&D), evaluation of binding activity against human EphA4 extracellular domain, EphA4 N-terminal fragments in human plasma, and EphA4 N-terminal fragments in human cerebrospinal fluid (CSF) was performed according to the following steps. KPEP11_10 was prepared to a final concentration of 1 µg/mL with 50 mM Tris-HCl (pH 7.5) /0.1% sodium azide, and coated onto the wells of a 96-well plate (Nunc) at 100 µL each. After incubating at 4°C overnight, the wells were blocked at room temperature for 1 hour or more or at 4°C overnight with a blocking solution (50 mM Tris-HCl (pH 7.5) /150 mM NaCl/0.01% Tween 20/5% skim milk (FUJIFILM Wako)). KPEP11_04, KPEP11_18, EphA4 antibody (Sino), and EphA4 antibody (R&D) were labeled with HRP with Peroxidase Labeling Kit-NH2 (DOJINDO) according to the attached manual. After washing the blocked plate three times with the wash solution (50 mM Tris-HCl (pH 7.5)/150 mM NaCl/0.01% Tween 20), the wells were seeded with each of human EphA4 extracellular domain serially diluted with the sample diluent (50 mM Tris-HCl (pH 7.5)/150 mM NaCl/0.2% EDTA-3Na/4% PEG 6000/0.01% Tween 20/0.2% Proclin 150 (Sigma-Aldrich)/5% skim milk (FUJIFILM Wako)) (0, 0.156, 0.313, 0.625, 1.25, 2.5, 5, and 10 ng/mL, shown as EphA4 in the Figure), diluted 50-fold human plasma, or diluted 100-fold human cerebrospinal fluid, and incubated at room temperature for 2 hours. After washing five times, HRP-labeled KPEP11_04, KPEP11_18, EphA4 antibody (Sino), or EphA4 antibody (R&D) diluted 50000-fold with the sample diluent was added at 100 µL each, and this was incubated at room temperature for 1 hour. After washing three times, TMBZ (3,3',5,5'-tetramethylbenzidine, KPL) solution was added to the wells, and this was incubated for 30 minutes at room temperature. Equal amounts of quenching solution (2 N H₂SO₄, FUJIFILM Wako) were added to the wells, and absorbance at 450 nm and 650 nm were read with a microplate reader (Molecular Device).

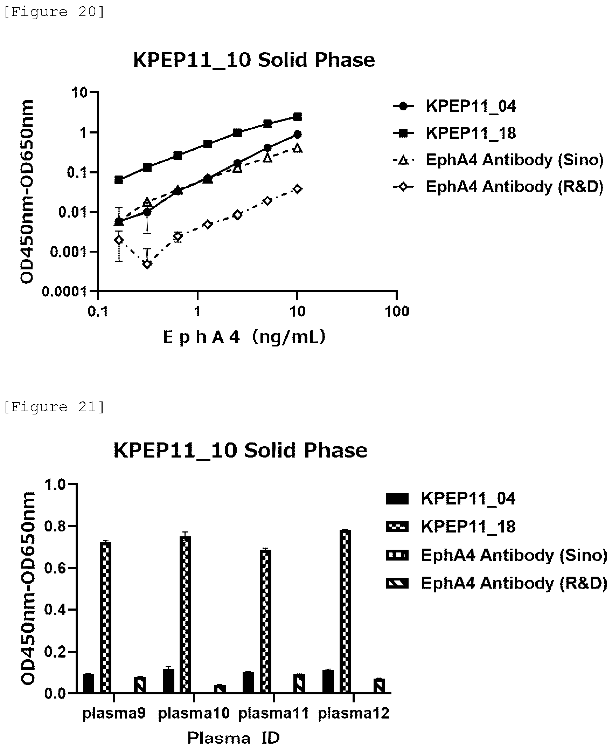

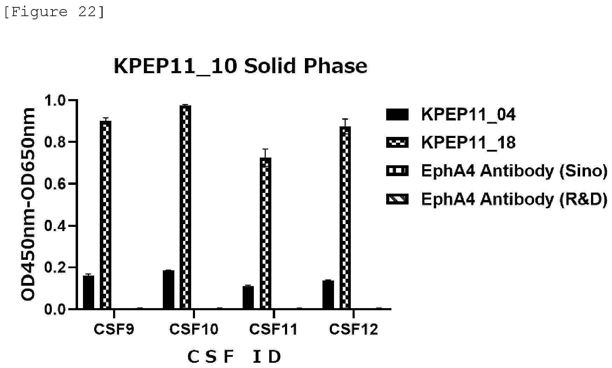

The result of evaluating reactivity against human EphA4 extracellular domain, the result of evaluating reactivity against EphA4 N-terminal fragments in human plasma, and the result of evaluating reactivity against EphA4 N-terminal fragments in human cerebrospinal fluid are shown in Figure 20, Figure 21, and Figure 22. Sandwich ELISA constructed with KPEP11_10 and HRP-labeled KPEP11_18 had extremely high reactivity against any of human EphA4 extracellular domain, EphA4 N-terminal fragments in human plasma, and EphA4 N-terminal fragments in human cerebrospinal fluid. Sandwich ELISA constructed with KPEP11_10 and HRP-labeled KPEP11_04 showed reactivity against any of human EphA4 extracellular domain, EphA4 N-terminal fragments in human plasma, and EphA4 N-terminal fragments in human cerebrospinal fluid.

### Example 15: Reactivity Against Human EphA4 Extracellular Domain, EphA4 N-Terminal Fragments in Human Plasma, and EphA4 N-Terminal Fragments in Human Cerebrospinal Fluid by Sandwich ELISA 6

For KPEP11_08, KPEP11_10, KPEP11_18, EphA4 antibody (Sino), and EphA4 antibody (R&D), evaluation of binding activity against human EphA4 extracellular domain, EphA4 N-terminal fragments in human plasma, and EphA4 N-terminal fragments in human cerebrospinal fluid (CSF) was performed according to the following steps. KPEP11 18 was prepared to a final concentration of 1 µg/mL with 50 mM Tris-HCl (pH 7.5)/0.1% sodium azide, and coated onto the wells of a 96-well plate (Nunc) at 100 µL each. After incubating at 4°C overnight, the wells were blocked at room temperature for 1 hour or more or at 4°C overnight with a blocking solution (50 mM Tris-HCl (pH 7.5)/150 mM NaCl/0.01% Tween 20/5% skim milk (FUJIFILM Wako)). KPEP11_08, KPEP11_10, EphA4 antibody (Sino), and EphA4 antibody (R&D) were labeled with HRP with Peroxidase Labeling Kit-NH2 (DOJINDO) according to the attached manual. After washing the blocked plate three times with the wash solution (50 mM Tris-HCl (pH 7.5)/150 mM NaCl/0.01% Tween 20), the wells were seeded with each of human EphA4 extracellular domain serially diluted with the sample diluent (50 mM Tris-HCl (pH 7.5)/150 mM NaCl/0.2% EDTA-3Na/4% PEG 6000/0.01% Tween 20/0.2% Proclin 150 (Sigma-Aldrich)/5% skim milk (FUJIFILMWako)) (0, 0.156, 0.313, 0.625, 1.25, 2.5, 5, and 10 ng/mL, shown as EphA4 in the Figure), diluted 50-fold human plasma, or diluted 100-fold human cerebrospinal fluid, and incubated at room temperature for 2 hours. After washing five times, HRP-labeled KPEP11_08, KPEP11_10, EphA4 antibody (Sino), or EphA4 antibody (R&D) diluted 50000-fold with the sample diluent was added at 100 µL each, and this was incubated at room temperature for 1 hour. After washing three times, TMBZ (3,3',5,5'-tetramethylbenzidine, KPL) solution was added to the wells, and this was incubated for 30 minutes at room temperature. Equal amounts of quenching solution (2 N H₂SO₄, FUJIFILM Wako) were added to the wells, and absorbance at 450 nm and 650 nm were read with a microplate reader (Molecular Device).

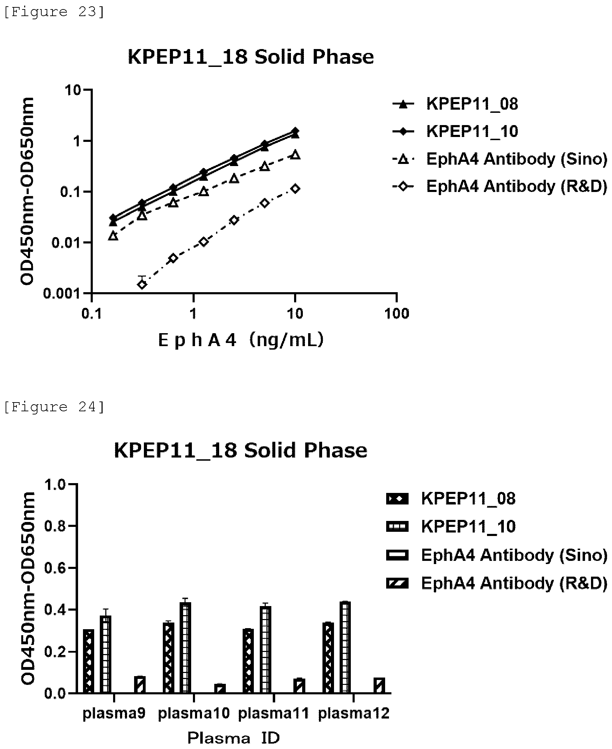

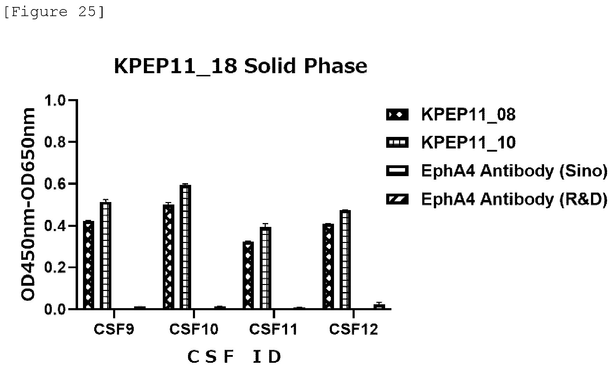

The result of evaluating reactivity against human EphA4 extracellular domain, the result of evaluating reactivity against EphA4 N-terminal fragments in human plasma, and the result of evaluating reactivity against EphA4 N-terminal fragments in human cerebrospinal fluid are shown in Figure 23, Figure 24, and Figure 25, respectively. Sandwich ELISA constructed with KPEP11_18 and HRP-labeled KPEP11_08 and sandwich ELISA constructed with KPEP11_18 and HRP-labeled KPEP11_10 had extremely high reactivity against any of human EphA4 extracellular domain, EphA4 N-terminal fragments in human plasma, and EphA4 N-terminal fragments in human cerebrospinal fluid.

### Example 16: Quantification Analysis and Correlation Analysis of EphA4 N-Terminal Fragments in Human Cerebrospinal Fluid by ELISA and LC-MS

### Quantification Analysis by LC-MS Analysis

Sample preparation was carried out as follows. As the sample for standard curve, 100 µL each of human EphA4 extracellular domain serially diluted with BSA (SIGMA) solution diluted to a final concentration of 500 µg/mL with aCSF (Harvard Apparatus) (0, 3.125, 6.25, 12.5, 25, 50, 100, and 200 ng/mL) was used. Fifty microliters of cerebrospinal fluid (CSF) sample were mixed with 50 µL of BSA (SIGMA) solution diluted to a final concentration of 500 µg/mL with aCSF (Harvard Apparatus), and then subjected to the following operations. One-hundred and fifty microliters of 10 M Urea solution dissolved in 150 µL of 50 mM TEAB (Thermo Fisher), further 25 µL of 100 mM DTT solution were sequentially added. After incubating the mixed solution at 37°C for 120 minutes, 25 µL of 200 mM iodoacetamide (FUJIFILM Wako) was added, and then this was incubated in the dark at room temperature for 30 minutes. Subsequently, 1250 µL of EphA4-NTF-IS and 10 µL of trypsin solution (200 µg/mL) were sequentially added, and this was incubated at 37°C for 18 hours. Eighty microliters of 20% TFA was added to quench trypsin digestion. Sample purification was performed as follows with Oasis HLB 96-Well Plate (Waters). After washing the plate with 500 µL of methanol (FUJIFILM Wako), this was equilibrated with 500 µL of 0.1% TFA (Thermo Fisher). After applying the sample prepared above and allowed to adsorb on the column, washing with 500 µL of 0.1% TFA was performed. Subsequently, 200 µL of the eluate (0.1% TFA-80% acetonitrile in water (FUJIFILM Wako)) was added, and the eluate was collected. The collected eluate was dried by SpeedVac system (Thermo Fisher), and subsequently, 30 µL of 0.1% TFA-5% acetonitrile in water was used to obtain the final reconstitution solution, and this solution was subjected to LC-MS analysis.

### Quantification Analysis by ELISA Analysis 1

Quantification analysis of EphA4 N-terminal fragments in human cerebrospinal fluid was performed according to the following steps. KPEP11 10 was prepared to a final concentration of 1 µg/mL with 50 mM Tris-HCl (pH 7.5)/0.1% sodium azide, and coated onto the wells of a 96-well plate (Nunc) at 100 µL each. After incubating at 4°C overnight, the wells were blocked at room temperature for 1 hour or more or at 4°C overnight with a blocking solution (50 mM Tris-HCl (pH 7.5)/150 mM NaCl/0.01% Tween 20/5% skim milk (FUJIFILM Wako)). KPEP11_18 was labeled with HRP with Peroxidase Labeling Kit-NH2 (DOJINDO) according to the attached manual. After washing the blocked plate three times with the wash solution (50 mM Tris-HCl (pH 7.5) /150 mM NaCl/0.01% Tween 20), the wells were seeded with each of human EphA4 extracellular domain serially diluted with the sample diluent (50 mM Tris-HCl (pH 7.5)/150 mM NaCl/0.2% EDTA-3Na/4% PEG 6000/0.01% Tween 20/0.2% Proclin 150 (Sigma-Aldrich)/5% skim milk (FUJIFILM Wako)) (0, 0.156, 0.313, 0.625, 1.25, 2.5, 5, and 10 ng/mL, shown as EphA4 in the Figure), or diluted 100-fold human cerebrospinal fluid, and incubated at room temperature for 2 hours. After washing five times, HRP-labeled KPEP11_18 diluted 50000-fold with the sample diluent was added at 100 µL each, and this was incubated at room temperature for 1 hour. After washing three times, TMBZ (3,3',5,5'-tetramethylbenzidine, KPL) solution was added to the wells, and this was incubated for 30 minutes at room temperature. Equal amounts of quenching solution (2 N H₂SO₄, FUJIFILM Wako) were added to the wells, and absorbance at 450 nm and 650 nm were read with a microplate reader (Molecular Device).

### Quantification Analysis by ELISA Analysis 2

Quantification analysis of EphA4 N-terminal fragments in human cerebrospinal fluid was performed according to the following steps. KPEP11 18 was prepared to a final concentration of 1 µg/mL with 50 mM Tris-HCl (pH 7.5)/0.1% sodium azide, and coated onto the wells of a 96-well plate (Nunc) at 100 µL each. After incubating at 4°C overnight, the wells were blocked at room temperature for 1 hour or more or at 4°C overnight with a blocking solution (50 mM Tris-HCl (pH 7.5)/150 mM NaCl/0.01% Tween 20/5% skim milk (FUJIFILM Wako)). KPEP11_10 was labeled with HRP with Peroxidase Labeling Kit-NH2 (DOJINDO) according to the attached manual. After washing the blocked plate three times with the wash solution (50 mM Tris-HCl (pH 7.5) /150 mM NaCl/0.01% Tween 20), the wells were seeded with each of human EphA4 extracellular domain serially diluted with the sample diluent (50 mM Tris-HCl (pH 7.5)/150 mM NaCl/0.2% EDTA-3Na/4% PEG 6000/0.01% Tween 20/0.2% Proclin 150 (Sigma-Aldrich)/5% skim milk (FUJIFILM Wako)) (0, 0.156, 0.313, 0.625, 1.25, 2.5, 5, and 10 ng/mL, shown as EphA4 in the Figure), or human cerebrospinal fluid diluted 100-fold, and this was incubated at room temperature for 2 hours. After washing five times, HRP-labeled KPEP11_10 diluted 40000-fold with the sample diluent was added at 100 µL each, and this was incubated at room temperature for 1 hour. After washing three times, TMBZ (3,3',5,5'-tetramethylbenzidine, KPL) solution was added to the wells, and this was incubated for 30 minutes at room temperature. Equal amounts of quenching solution (2 N H₂SO₄, FUJIFILM Wako) were added to the wells, and absorbance at 450 nm and 650 nm were read with a microplate reader (Molecular Device).

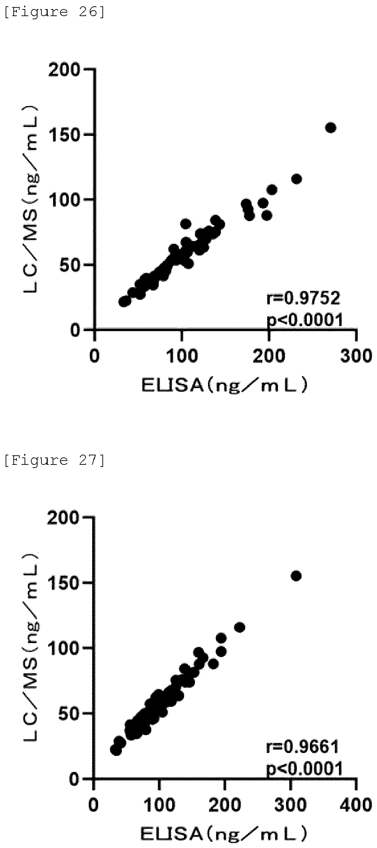

The quantification result of EphA4 N-terminal fragments in human cerebrospinal fluid analyzed with the quantification result of EphA4 N-terminal fragments in human cerebrospinal fluid analyzed by LC-MS, as well as the correlation analysis result carried out based on Quantification analysis by ELISA analysis 1 are shown in Figure 26. The Spearman correlation coefficient (r) was calculated between the amount of EphA4 N-terminal fragments quantified by LC-MS 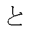 the amount of EphA4 N-terminal fragments quantified by ELISA, and it was found that a significant correlation was seen between them (p < 0.0001).

The quantification result of EphA4 N-terminal fragments in human cerebrospinal fluid analyzed by LC-MS, as well as the correlation analysis result carried out based on Quantification analysis by ELISA analysis 2 are shown in Figure 27. The Spearman correlation coefficient (r) was calculated between the amount of EphA4 N-terminal fragments quantified by LC-MS and the amount of EphA4 N-terminal fragments quantified by ELISA, and it was found that a significant correlation was seen between them (p < 0.0001).

## Claims

1. An anti-EphA4 antibody or an antigen binding fragment thereof, wherein the antibody comprises
(a) a heavy chain comprising a heavy chain CDR1 consisting of the amino acid sequence shown in SEQ ID NO. 52; a heavy chain CDR2 consisting of the amino acid sequence shown in SEQ ID NO. 53; and a heavy chain CDR3 consisting of the amino acid sequence shown in SEQ ID NO. 54; and
a light chain comprising a light chain CDR1 consisting of the amino acid sequence shown in SEQ ID NO. 55; a light chain CDR2 consisting of the amino acid sequence shown in SEQ ID NO. 56; and a light chain CDR3 consisting of the amino acid sequence shown in SEQ ID NO. 57;
(b) a heavy chain comprising a heavy chain CDR1 consisting of the amino acid sequence shown in SEQ ID NO. 64; a heavy chain CDR2 consisting of the amino acid sequence shown in SEQ ID NO. 65; and a heavy chain CDR3 consisting of the amino acid sequence shown in SEQ ID NO. 66; and
a light chain comprising a light chain CDR1 consisting of the amino acid sequence shown in SEQ ID NO. 67; a light chain CDR2 consisting of the amino acid sequence shown in SEQ ID NO. 68; and a light chain CDR3 consisting of the amino acid sequence shown in SEQ ID NO. 69; or
(c) a heavy chain comprising a heavy chain CDR1 consisting of the amino acid sequence shown in SEQ ID NO. 40; a heavy chain CDR2 consisting of the amino acid sequence shown in SEQ ID NO. 41; and a heavy chain CDR3 consisting of the amino acid sequence shown in SEQ ID NO. 42; and
a light chain comprising a light chain CDR1 consisting of the amino acid sequence shown in SEQ ID NO. 43; a light chain CDR2 consisting of the amino acid sequence shown in SEQ ID NO. 44; and a light chain CDR3 consisting of the amino acid sequence shown in SEQ ID NO. 45.

2. The anti-EphA4 antibody or antigen binding fragment thereof according to claim 1, wherein the antibody comprises
a heavy chain comprising a heavy chain CDR1 consisting of the amino acid sequence shown in SEQ ID NO. 52; a heavy chain CDR2 consisting of the amino acid sequence shown in SEQ ID NO. 53; and a heavy chain CDR3 consisting of the amino acid sequence shown in SEQ ID NO. 54; and
a light chain comprising a light chain CDR1 consisting of the amino acid sequence shown in SEQ ID NO. 55; a light chain CDR2 consisting of the amino acid sequence shown in SEQ ID NO. 56; and a light chain CDR3 consisting of the amino acid sequence shown in SEQ ID NO. 57.

3. The anti-EphA4 antibody or antigen binding fragment thereof according to claim 2, wherein the antibody comprises
a heavy chain variable region consisting of the amino acid sequence shown in SEQ ID NO. 10, and
a light chain variable region consisting of the amino acid sequence shown in SEQ ID NO. 11.

4. The anti-EphA4 antibody or antigen binding fragment thereof according to claim 2, wherein the antibody comprises
a heavy chain variable region consisting of the amino acid sequence shown in SEQ ID NO. 14, and
a light chain variable region consisting of the amino acid sequence shown in SEQ ID NO. 15.

5. The anti-EphA4 antibody or antigen binding fragment thereof according to claim 1, wherein the antibody comprises
a heavy chain comprising a heavy chain CDR1 consisting of the amino acid sequence shown in SEQ ID NO. 64; a heavy chain CDR2 consisting of the amino acid sequence shown in SEQ ID NO. 65; and a heavy chain CDR3 consisting of the amino acid sequence shown in SEQ ID NO. 66; and
a light chain comprising a light chain CDR1 consisting of the amino acid sequence shown in SEQ ID NO. 67; a light chain CDR2 consisting of the amino acid sequence shown in SEQ ID NO. 68; and a light chain CDR3 consisting of the amino acid sequence shown in SEQ ID NO. 69.

6. The anti-EphA4 antibody or antigen binding fragment thereof according to claim 5, wherein the antibody comprises
a heavy chain variable region consisting of the amino acid sequence shown in SEQ ID NO. 18, and
a light chain variable region consisting of the amino acid sequence shown in SEQ ID NO. 19.

7. The anti-EphA4 antibody or antigen binding fragment thereof according to claim 1, wherein the antibody comprises
a heavy chain comprising a heavy chain CDR1 consisting of the amino acid sequence shown in SEQ ID NO. 40; a heavy chain CDR2 consisting of the amino acid sequence shown in SEQ ID NO. 41; and a heavy chain CDR3 consisting of the amino acid sequence shown in SEQ ID NO. 42; and
a light chain comprising a light chain CDR1 consisting of the amino acid sequence shown in SEQ ID NO. 43; a light chain CDR2 consisting of the amino acid sequence shown in SEQ ID NO. 44; and a light chain CDR3 consisting of the amino acid sequence shown in SEQ ID NO. 45.

8. The anti-EphA4 antibody or antigen binding fragment thereof according to claim 7, wherein the antibody comprises
a heavy chain variable region consisting of the amino acid sequence shown in SEQ ID NO. 6, and
a light chain variable region consisting of the amino acid sequence shown in SEQ ID NO. 7.

9. The anti-EphA4 antibody or antigen binding fragment thereof according to any one of claims 1 to 8, wherein the antibody or an antigen binding fragment thereof is labeled.

10. A method for producing an anti-EphA4 antibody or an antigen binding fragment thereof, comprising a step of culturing a host cell comprising an isolated nucleic acid encoding the anti-EphA4 antibody or antigen binding fragment thereof according to any one of claims 1 to 8.

11. A method for detecting or quantifying human EphA4 in a biological sample, comprising contacting the biological sample with the anti-EphA4 antibody or antigen binding fragment thereof according to any one of claims 1 to 8.

12. The method according to claim 11, wherein the human EphA4 is the N-terminal fragment of human EphA4.

13. The method according to claim 11 or 12, wherein the biological sample is blood, serum, plasma, or cerebrospinal fluid.

14. The method according to any one of claims 11 to 13, further comprising contacting the biological sample with the labeled anti-EphA4 antibody or antigen binding fragment thereof according to claim 9.

15. A kit for detecting or quantifying human EphA4, comprising the anti-EphA4 antibody or antigen binding fragment thereof according to any one of claims 1 to 9.

16. The kit according to claim 15, wherein the human EphA4 is the N-terminal fragment of human EphA4.

17. The kit according to claim 15 or 16, comprising
an anti-EphA4 antibody or an antigen binding fragment thereof comprising a heavy chain variable region consisting of the amino acid sequence shown in SEQ ID NO. 14 and a light chain variable region consisting of the amino acid sequence shown in SEQ ID NO. 15, and
an anti-EphA4 antibody or an antigen binding fragment thereof comprising a heavy chain variable region consisting of the amino acid sequence shown in SEQ ID NO. 18 and a light chain variable region consisting of the amino acid sequence shown in SEQ ID NO. 19.

18. The kit according to claim 15 or 16, comprising
an anti-EphA4 antibody or an antigen binding fragment thereof comprising a heavy chain variable region consisting of the amino acid sequence shown in SEQ ID NO. 6 and a light chain variable region consisting of the amino acid sequence shown in SEQ ID NO. 7, and
an anti-EphA4 antibody or an antigen binding fragment thereof comprising a heavy chain variable region consisting of the amino acid sequence shown in SEQ ID NO. 14 and a light chain variable region consisting of the amino acid sequence shown in SEQ ID NO. 15.

19. The kit according to claim 15 or 16, comprising
an anti-EphA4 antibody or an antigen binding fragment thereof comprising a heavy chain variable region consisting of the amino acid sequence shown in SEQ ID NO. 10 and a light chain variable region consisting of the amino acid sequence shown in SEQ ID NO. 11, and
an anti-EphA4 antibody or an antigen binding fragment thereof comprising a heavy chain variable region consisting of the amino acid sequence shown in SEQ ID NO. 18 and a light chain variable region consisting of the amino acid sequence shown in SEQ ID NO. 19.

20. The kit according to any one of claims 15 to 19, wherein the kit comprises the N-terminal fragment of human EphA4.
